# EUROPEAN PATENT APPLICATION

(11) **EP 4 847 619 A1**
(43) Date of publication of application: **02.12.2026**
(21) Application number: 25745051.0
(22) Date of filing: 21.01.2025
(51) Int. Cl.: C12N 15/19, A61K 35/76, A61K 35/761, A61K 48/00, A61P 35/00, A61P 35/04, A61P 43/00, C12N 7/01, C12N 15/26, C12N 15/27, C12N 15/34

(54) **ONCOLYTIC VIRUS AND CANCER TREATMENT USING SAME**

(30) Priority: 22.01.2024 JP 2024007682
(71) Applicant: Kagoshima University, Kagoshima-shi, Kagoshima 890-8580 (JP); Surv Biopharma Inc., Kagoshima-shi, Kagoshima, 890-8544 (JP)
(72) Inventor: KOSAI, Ken-ichiro, Kagoshima-shi, Kagoshima 890-8580 (JP); WATANABE, Maki, Kagoshima-shi, Kagoshima 890-8580 (JP); MATSUDA, Eriko, Kagoshima-shi, Kagoshima 890-8580 (JP); NISHIKAWAJI, Yuya, Kagoshima-shi, Kagoshima 890-8580 (JP)
(74) Representative: Weickmann & Weickmann PartmbB
(86) International application number: PCT/JP2025/001686
(87) International publication number: WO 2025/159067

(57) **Abstract**

The present invention provides an oncolytic virus such as a conditionally replicating adenovirus carrying the CXCL10 gene, the oncolytic virus further carrying the IL-2 gene and/or GM-CSF gene on the same viral genome, a combination of the oncolytic virus and another oncolytic virus carrying the IL-2 gene and/or GM-CSF gene, and a cancer therapeutic agent containing any of the oncolytic viruses or combinations as an active ingredient.

## Description

### [Technical Field]

The present invention relates to an oncolytic virus carrying immunostimulatory gene(s) including CXC motif chemokine ligand 10 (CXCL10) and use thereof in cancer treatment. More particularly, the present invention relates to an oncolytic virus carrying the CXCL10 gene, an oncolytic virus containing two or three immunostimulatory genes including the CXCL10 gene, wherein the CXCL10 gene and the interleukin-2 (IL-2) and/or granulocyte-macrophage-colony-stimulating factor (GM-CSF) gene are carried in the same or separate oncolytic viruses, and to cancer treatment using these, particularly effective treatment for invasive and metastatic refractory cancers.

### [Background Art]

In recent years, conditionally replicating adenovirus (CRA), which specifically replicates in cancer cells and kills the same, has been considered promising in cancer treatment. CRA was created by modifying the E1 gene region, which is essential for adenovirus replication, to induce differences in viral replication between cancer cells and normal cells. It includes two types: (1) one that inhibits the inactivation of Rb and p53, which are essential for inducing the cellular environment necessary for adenovirus replication, by deleting binding regions binding thereto within the E1 region (i.e., preventing viral replication in normal cells) and (2) one that cancer-specifically expresses the E1 gene (causes cancer-specific viral replication) by replacing the endogenous promoter of the E1 gene with the promoter of a gene that is highly expressed specifically in cancer.

The present inventors have developed a technology enabling efficient production of a "CRA which can be controlled and treated through multiple factors" (m-CRA), which can be called a next-generation CRA, and capable of precise control of viral replication and enhancement of treatment effects through numerous cancer-specific factors and therapeutic genes, such as deletion within the E1A and E1B gene regions, substitution of the endogenous promoter of the gene with an exogenous promoter, and further incorporation of other cancer therapeutic genes (Patent Literature 1, Non Patent Literature 1). Furthermore, using this technology, they have produced an m-CRA (Surv.m-CRA-1) in which the virus proliferates under the control of the promoter of survivin, which is in the inhibitor of apoptosis protein (IAP) family, and have investigated and reported anticancer actions against various cancers that specifically express survivin (Patent Literatures 2, 3, Non Patent Literature 2).

The greatest characteristic by which viral therapy differs from chemotherapy and radiation therapy is that, in the process in which amplified viruses destroy cancer cells, antitumor immunity centered on cellular immunity is induced by the release of tumor-associated antigens and the adjuvant-like function of the viruses, and treatment effects can be exerted throughout the body via immunity. That is, it is considered that, in the process in which the immune system eliminates the viruses, tumor-associated antigens derived from destroyed cancer cells are processed and presented by antigen-presenting cells, and cancer-specific immunity is elicited. In order to maximize this antitumor immunity, oncolytic viral immunotherapy (OVI) using oncolytic viruses carrying immunostimulatory genes is being actively studied. For example, talimogene laherparepvec, which is the only OVI approved in Europe and the United States, is an oncolytic herpes simplex virus carrying a GM-CSF gene as an immunostimulatory gene downstream of a ubiquitous and strong CMV promoter.

Generally, the development of oncolytic adenoviruses containing cytokine genes is hindered by two technical limitations: vector construction and animal models that allow viral replication. The present inventors efficiently produced oncolytic adenoviruses carrying various candidate immunostimulatory genes under the control of various candidate promoters using m-CRA technology, established an immunocompetent syngeneic hamster cancer model in which human adenoviruses can replicate, and used this model to establish an evaluation system for systematically and accurately analyzing their *in vivo* treatment effects (Non Patent Literature 3). Using this evaluation system, they tested m-CRAs (Surv.m-CRA-2-G) in which a GM-CSF gene was ligated downstream of various promoters, and revealed that they could be safely administered while maintaining treatment effects (Patent Literature 4). Furthermore, they clarified that an m-CRA carrying a GM-CSF gene showed survival prolongation equivalent to or greater than that of an anti-PD-1 antibody, which is one of the existing excellent cancer immunotherapeutic agents (Patent Literature 4).

However, the cancer treatment effect of oncolytic viruses carrying the GM-CSF gene may not always be sufficient. In particular, there is a need for further improvement in treatment effect against invasive and metastatic refractory cancers.

### [Citation List]

### [Patent Literature]

[Patent Literature 1]
   JP 2005-46101 A
[Patent Literature 2]
   WO 2005/115476
[Patent Literature 3]
   WO 2022/244792
[Patent Literature 4]
   WO 2019/093435

### [Non Patent Literature]

[Non Patent Literature 1]
   Nagano, S. et al., Gene Ther., 2005, 12(18): 1385-93
[Non Patent Literature 2]
   Kamizono, J. et al., Cancer Res., 2005, 65(12): 5284-91
[Non Patent Literature 3]
   Ijichi, N. et al., Mol. Ther., 2016, 24(S1): S164

### [Summary of Invention]

### [Technical Problem]

The object of the present invention is to provide a novel OVI having not only a cancer treatment effect, particularly a tumor growth suppressive effect in a primary lesion, but also further enhanced systemic antitumor immunity, which is important for a treatment effect in metastatic lesions.

### [Solution to Problem]

The present inventors have conducted intensive studies in an attempt to achieve the above-mentioned object and found that when the CXCL10 gene is used as the immunostimulatory gene, the OVI carrying this gene specifically damages various cancer cells more efficiently than oncolytic viruses that do not contain immunogenes, and can remarkably suppress tumor growth even when administered locally to a tumor-bearing hamster model.

Even when OVIs carrying the CXCL10 or GM-CSF gene did not show a significant growth suppressive effect as a single agent due to differences in the conditions such as tumor types and corresponding dosages, the combined use of two agents of OVI carrying the CXCL10 gene and OVI carrying the GM-CSF gene or IL-2 gene could remarkably suppress tumor growth. In addition, when IL-2 gene was used as the central component and combined with CXCL10 and/or GM-CSF genes, OVI carrying the IL-2 gene tended to suppress tumor growth when used alone compared with oncolytic viruses without immunogenes. The combined use of two agents of IL-2 and CXCL10 or GM-CSF genes further enhanced the effect, and the combined use of three agents significantly suppressed tumor growth compared with use of oncolytic viruses without immunogenes.

Furthermore, distant site challenge tests of primary cancer cells and heterologous cancer cells revealed the induction of primary cancer cell-specific systemic antitumor immunity, where the treatment effect in distant metastatic lesions became higher in the order of IL-2 gene alone, combined use of two agents, and combined use of three agents, as in the case of primary lesion.

The weight gain impairment due to the introduction of immunostimulatory genes was mild in all cases, suggesting the absence of major side effects even when two factors or three factors are used in combination.

In addition, the present inventors designed and constructed a multifactor-carrying OVI capable of expressing two factors dicistronically or three factors tricistronically, in an attempt to improve the efficiency of co-introduction and co-expression of two or more immunostimulatory genes and to reduce the viral dose. The three-factor-carrying OVI suppressed the proliferation of primary tumors to a similar extent in a tumor-bearing hamster model, compared with a cocktail of three respective OVIs, carrying each gene, at the same total dose, and surprisingly, more strongly inhibited distant metastasis. The OVI carrying three therapeutic genes derived from human and mouse suppressed both primary cancer and metastatic cancer more markedly than the OVI without therapeutic genes.

Based on these findings, the present inventors conducted further studies and completed the present invention. That is, the present invention provides the following.

### [Item 1]

An oncolytic virus comprising a nucleic acid encoding CXC motif chemokine ligand 10 (CXCL10) under the control of a promoter functional in cancer cells.

### [Item 2]

The oncolytic virus of Item 1, wherein the aforementioned promoter is a ubiquitous promoter, a cancer cell-specific promoter, or an organ-specific promoter of the organ from which the cancer cells are derived.

### [Item 3]

The oncolytic virus of Item 1, wherein the aforementioned promoter is a CA promoter, a CMV promoter, an RSV promoter, or an E2F promoter.

### [Item 4]

The oncolytic virus of any one of Items 1 to 3, wherein the promoter of a nucleic acid encoding at least one factor essential for viral replication or assembly is substituted with a cancer cell-specific promoter or an organ-specific promoter of the organ from which the cancer cells are derived.

### [Item 5]

The oncolytic virus of Item 4, wherein the cancer cell-specific promoter is a survivin promoter.

### [Item 6]

The oncolytic virus of Item 4 or 5, wherein the virus is an adenovirus.

### [Item 7]

The oncolytic virus of Item 6, wherein the factor essential for viral replication or assembly is E1A or E1AΔ24, and/or E1B or E1BΔ55K.

### [Item 8]

The oncolytic virus of Item 7, wherein the promoter of the nucleic acid encoding E1A is substituted with a survivin promoter, and the promoter of the nucleic acid encoding E1BΔ55K is further substituted with an exogenous promoter selected from a ubiquitous promoter, a cancer cell-specific promoter, and an organ-specific promoter of the organ from which the cancer cells are derived.

### [Item 9]

The oncolytic virus of Item 8, wherein the promoter of the nucleic acid encoding E1BΔ55K is substituted with a CMV promoter.

### [Item 10]

The oncolytic virus of any one of Items 1 to 9, further comprising a nucleic acid encoding interleukin-2 (IL-2) under the control of a promoter functional in cancer cells, and/or a nucleic acid encoding granulocyte-macrophage-colony-stimulating factor (GM-CSF) under the control of a promoter functional in cancer cells.

### [Item 11]

The oncolytic virus of any one of Items 1 to 10, further comprising a nucleic acid encoding interleukin-2 (IL-2) under the control of a promoter functional in cancer cells, and a nucleic acid encoding granulocyte-macrophage colony-stimulating factor (GM-CSF) under the control of a promoter functional in cancer cells.

### [Item 12]

The oncolytic virus of Item 10 or 11, wherein the promoter controlling expression of CXCL10 and the promoter controlling expression of IL-2 and/or the promoter controlling expression of GM-CSF are the same single promoter.

### [Item 13]

The oncolytic virus of Item 12, wherein the nucleic acid encoding CXCL10 and the nucleic acid encoding IL-2 and/or the nucleic acid encoding GM-CSF are arranged in the following order from the 5' end:
(i) CXCL10-IL-2;
(ii) CXCL10-GM-CSF;
(iii) IL-2-CXCL10;
(iv) GM-CSF-CXCL10;
(v) CXCL10-IL-2-GM-CSF;
(vi) CXCL10-GM-CSF-IL-2;
(vii) IL-2-CXCL10-GM-CSF;
(viii) IL-2-GM-CSF-CXCL10;
(ix) GM-CSF-CXCL10-IL-2; or
(x) GM-CSF-IL-2-CXCL10.

### [Item 14]

The oncolytic virus of Item 13, wherein each of the aforementioned nucleic acids is linked via a 2A sequence or an IRES sequence.

### [Item 15]

The oncolytic virus of Item 14, wherein, from the 5' end, the first nucleic acid and the second nucleic acid are linked via a P2A sequence, and the second nucleic acid and the third nucleic acid are linked via a T2A sequence.

### [Item 16]

A combination of the oncolytic virus of any one of Items 1 to 9 and the oncolytic viruses of (a) and/or (b), or the oncolytic virus of (c) below:
(a) an oncolytic virus comprising a nucleic acid encoding IL-2 under the control of a promoter functional in cancer cells
(b) an oncolytic virus comprising a nucleic acid encoding GM-CSF under the control of a promoter functional in cancer cells
(c) an oncolytic virus comprising a nucleic acid encoding IL-2 under the control of a promoter functional in cancer cells, and a nucleic acid encoding GM-CSF under the control of a promoter functional in cancer cells.

### [Item 17]

The combination of Item 16, wherein the aforementioned promoters functional in cancer cells are the same or different, and each is a ubiquitous promoter, a cancer cell-specific promoter, or an organ-specific promoter of the organ from which the cancer cells are derived.

### [Item 18]

The combination of Item 16, wherein the aforementioned promoters functional in cancer cells are identical or different, and each is a CA promoter, a CMV promoter, an RSV promoter, or an E2F promoter.

### [Item 19]

The combination of any one of Items 16 to 18, wherein the oncolytic virus is a conditionally replicating adenovirus, and the structure of the replication control region of each oncolytic virus is identical.

### [Item 20]

A cancer therapeutic agent comprising the oncolytic virus of any one of Items 1 to 15 or the combination of any one of Items 16 to 19 as an active ingredient.

### [Item 20a]

A method for treating cancer, comprising administering an effective amount of the oncolytic virus of any one of Items 1 to 15 or the combination of any one of Items 16 to 19 to a subject having cancer.

### [Item 20b]

The oncolytic virus of any one of Items 1 to 15 or the combination of any one of Items 16 to 19, for use in the treatment of cancer.

### [Item 20c]

Use of the oncolytic virus of any one of Items 1 to 15 or the combination of any one of Items 16 to 19, for the manufacture of a cancer therapeutic agent.

### [Item 21]

The agent of Item 20, for local administration to a primary cancer lesion.

### [Item 22]

The agent of Item 20 or 21, for the treatment of invasive or metastatic cancer.

### [Item 23]

The agent of any one of Items 20 to 22, for multiple administration.

### [Advantageous Effects of Invention]

According to the present invention, cell-mediated systemic antitumor immunity specific to cancer antigens released from tumor cells killed by replication of an oncolytic virus can be induced to a higher level than by conventional techniques. Consequently, in addition to enhancing treatment effect at the primary lesion, the invention may provide an effective therapeutic means for invasive and metastatic refractory cancers.

### [Brief Description of Drawings]

[Fig. 1-1]

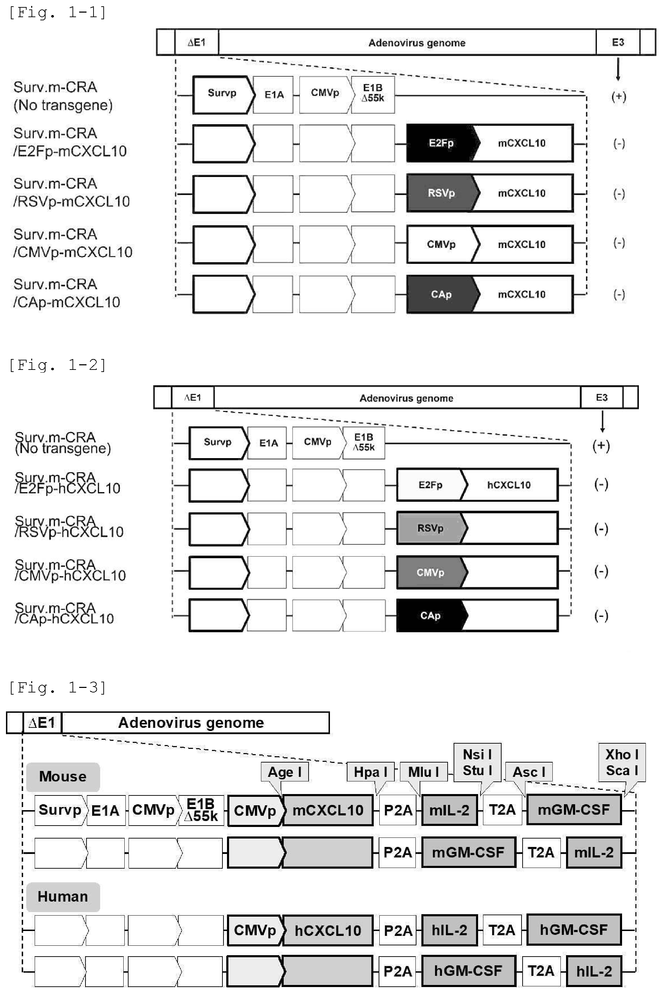

   Fig. 1-1 is a schematic diagram of the genome sequences of various Surv.m-CRAs having a unit of a mouse-derived CXCL10 gene downstream of various promoters produced in Example 1.
[Fig. 1-2]
   Fig. 1-2 is a schematic diagram of the genome sequences of various Surv.m-CRAs having a unit of a human-derived CXCL10 gene downstream of various promoters produced in Example 1.
[Fig. 1-3]
   Fig. 1-3 is a schematic diagram of the genome sequences of those including three therapeutic genes among the various Surv.m-CRAs produced in Example 1.
[Fig. 2-1]

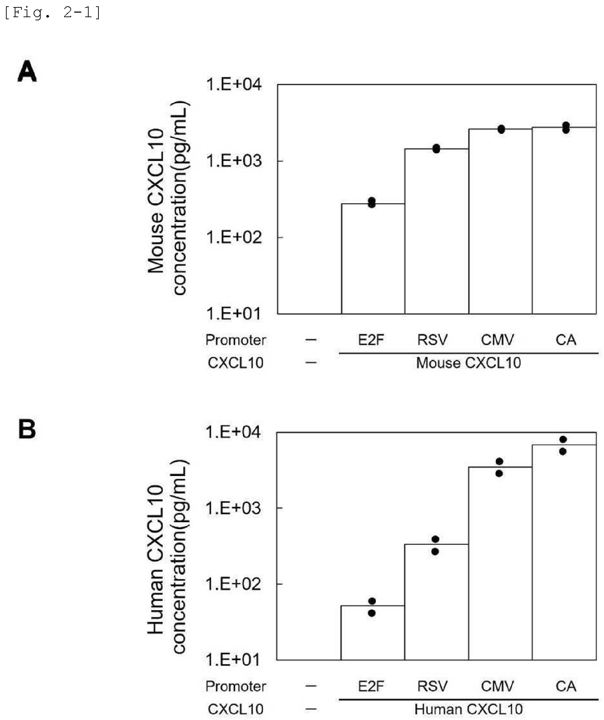

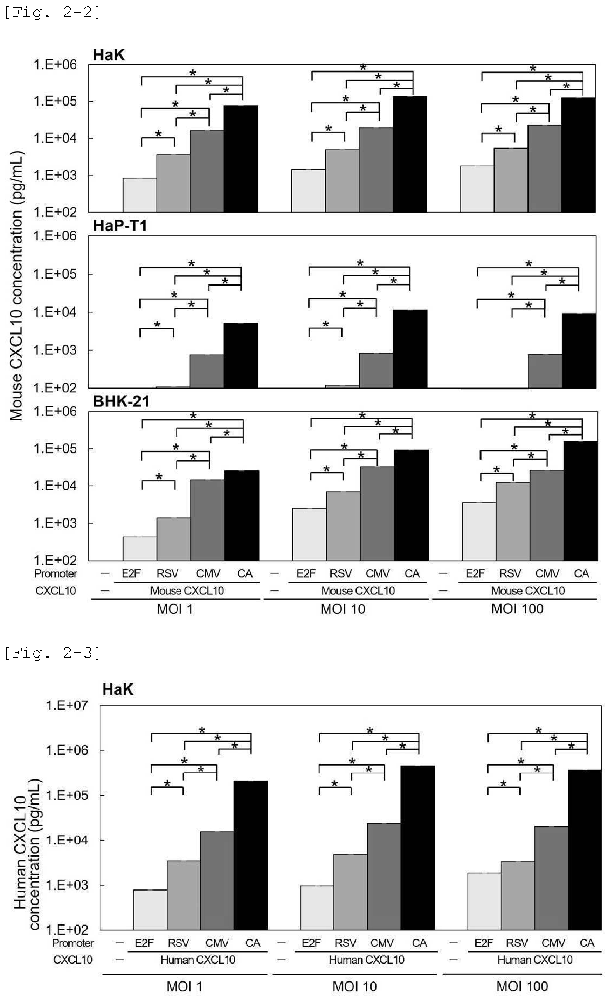

   Fig. 2-1 is a figure showing results of quantifying, using ELISA, mouse or human CXCL10 in culture supernatants of HEK293 cells transfected with a P2 plasmid including a nucleic acid encoding mouse CXCL10. (A: mouse CXCL10, B: human CXCL10)
[Fig. 2-2]
   Fig. 2-2 is a figure showing results of quantifying, using ELISA, expression of mouse CXCL10 in HaK cells, HaP-T1 cells, or BHK-21 cells infected with CRA carrying a mouse CXCL10 gene. *P<0.01
[Fig. 2-3]
   Fig. 2-3 is a figure showing results of quantifying, using ELISA, expression of human CXCL10 in HaK cells infected with CRA carrying a human CXCL10 gene. *P<0.01
[Fig. 3-1]

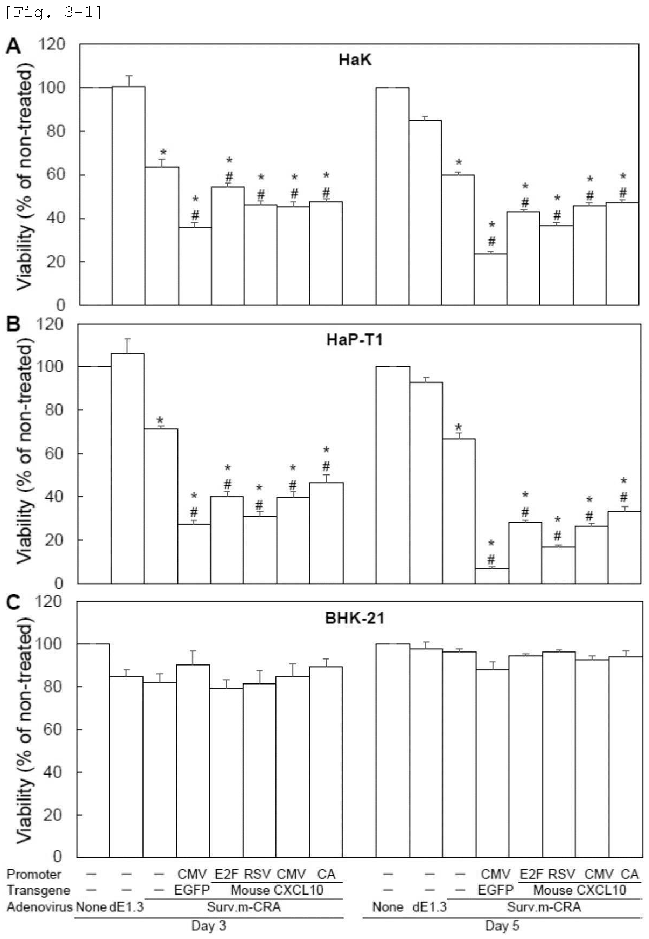

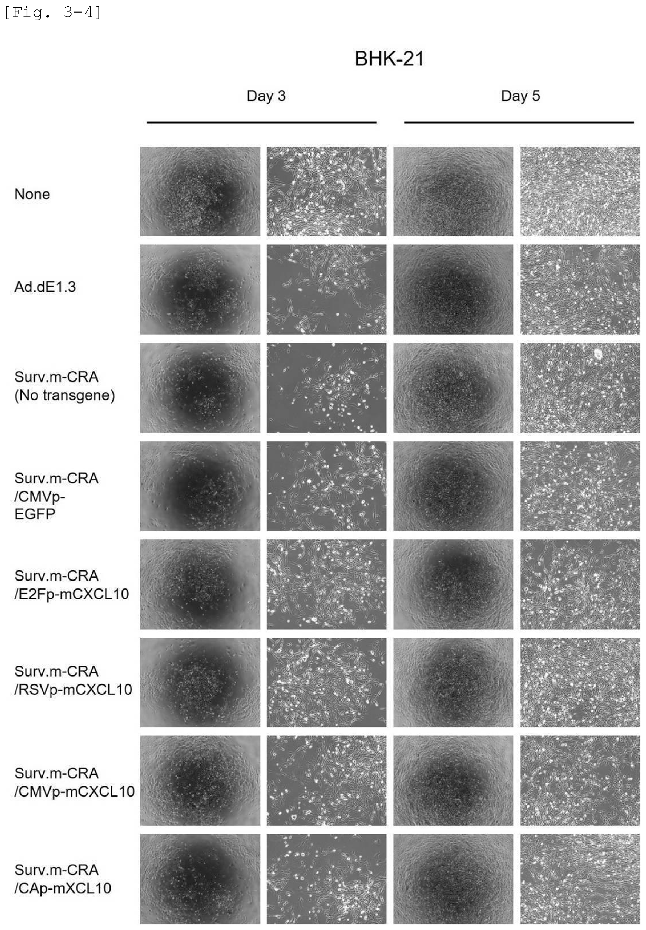

   Fig. 3-1 is a figure showing cytotoxic effects after *in vitro* infection with various Surv.m-CRAs in Example 3. *P<0.05 (vs. Ad.dE1.3); #P<0.05 (Surv.m-CRA (No transgene)).
[Fig. 3-2]

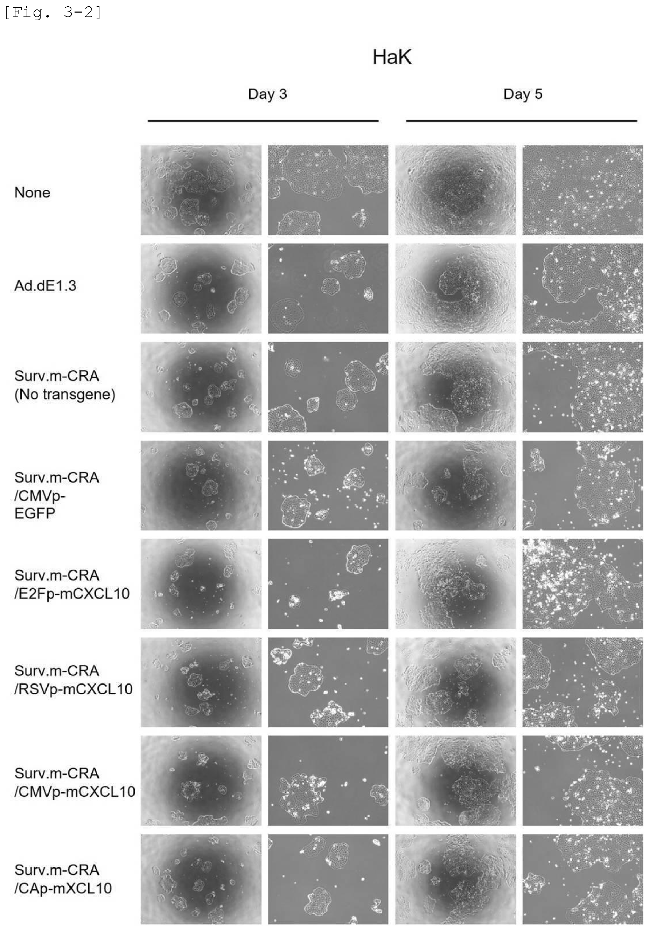

   Fig. 3-2 shows representative phase-contrast images of HaK cells at 3 days or 5 days after infection in Example 3, at magnifications of 40× (left) and 100× (right).
[Fig. 3-3]

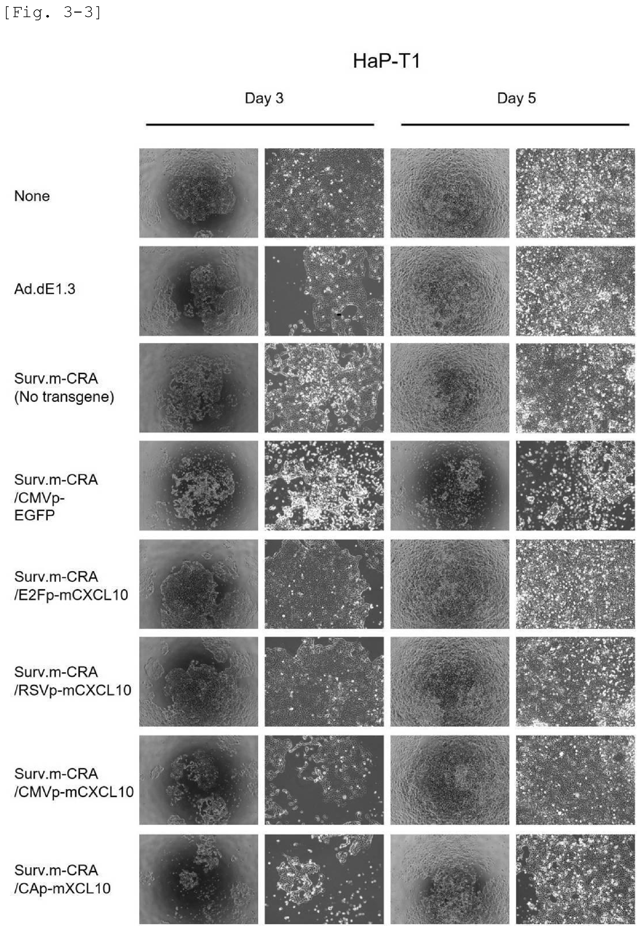

   Fig. 3-3 shows representative phase-contrast images of HaP-T1 cells at 3 days or 5 days after infection in Example 3, at magnifications of 40× (left) and 100× (right).
[Fig. 3-4]
   Fig. 3-4 shows representative phase-contrast images of BHK-21 cells at 3 days or 5 days after infection in Example 3, at magnifications of 40× (left) and 100× (right).
[Fig. 4]

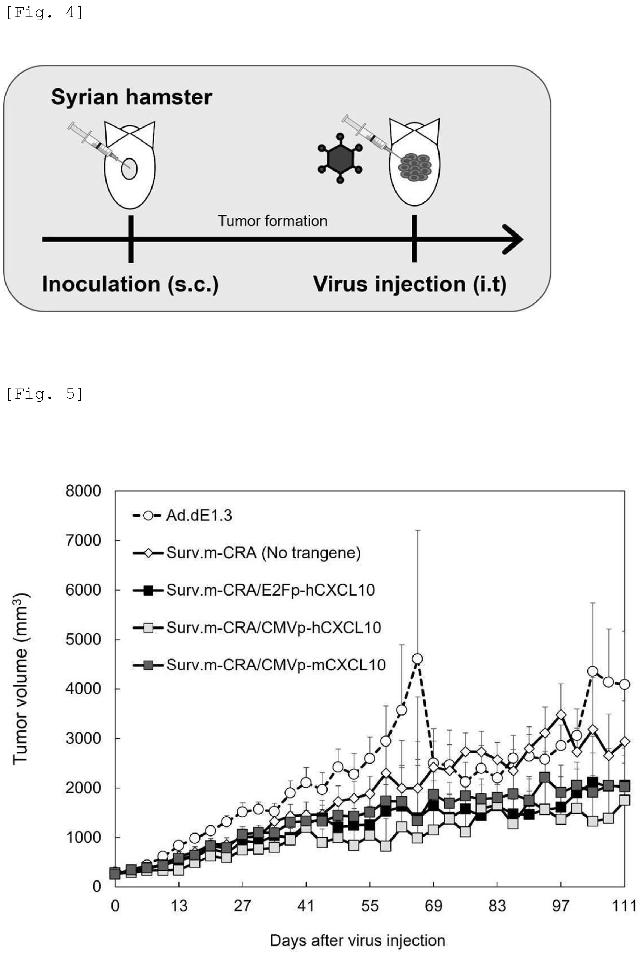

   Fig. 4 is a figure outlining the establishment process of a syngeneic Syrian hamster cancer model in Example 4.
[Fig. 5]
   Fig. 5 is a figure showing *in vivo* treatment effects of Surv.m-CRA carrying CXCL10 against subcutaneous tumors in syngeneic Syrian hamsters in Example 5.
[Fig. 6]

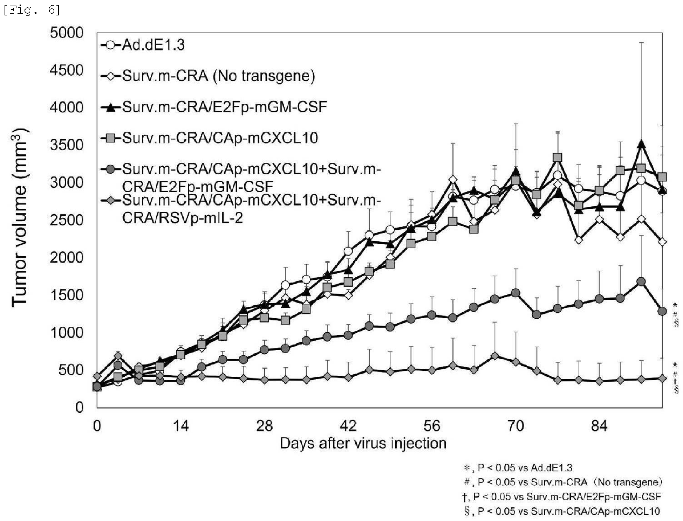

   Fig. 6 is a figure showing Surv.m-CRA-2 treatment effects by combined use of two cytokines in Example 6.
[Fig. 7-1]

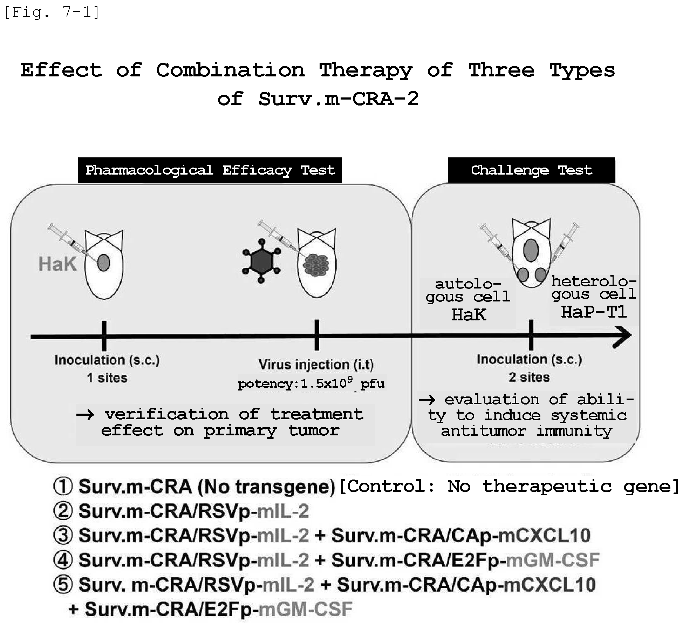

   Fig. 7-1 is a figure showing an outline of the experimental protocol in Example 7.
[Fig. 7-2]

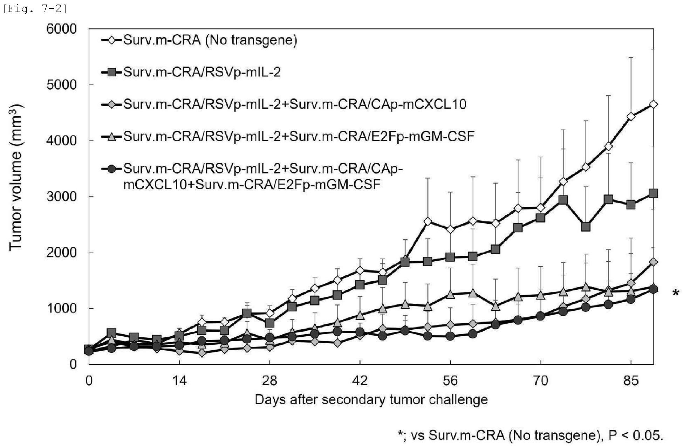

   Fig. 7-2 is a figure showing treatment effects on primary tumors by combined use of three types of Surv.m-CRA-2 in Example 7.
[Fig. 7-3]

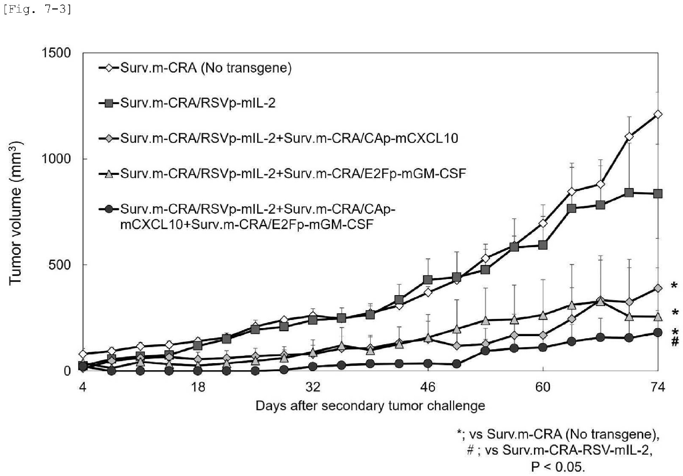

   Fig. 7-3 is a figure confirming regression of distant-site HaK tumors by combination therapy of three types of Surv.m-CRA-2 in Example 7.
[Fig. 7-4]

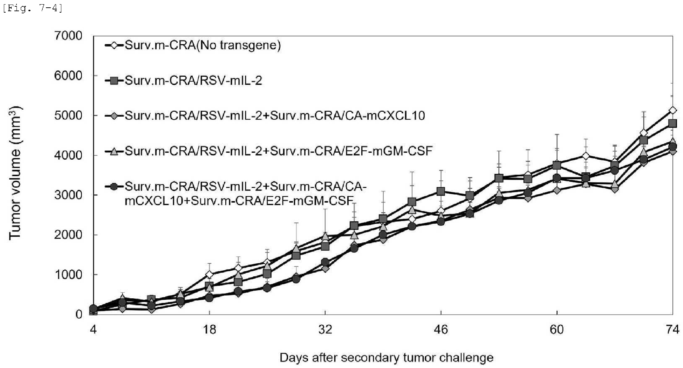

   Fig. 7-4 is a figure confirming regression of distant-site HaP-T1 tumors by combination therapy of three types of Surv.m-CRA-2 in Example 7.
[Fig. 7-5]

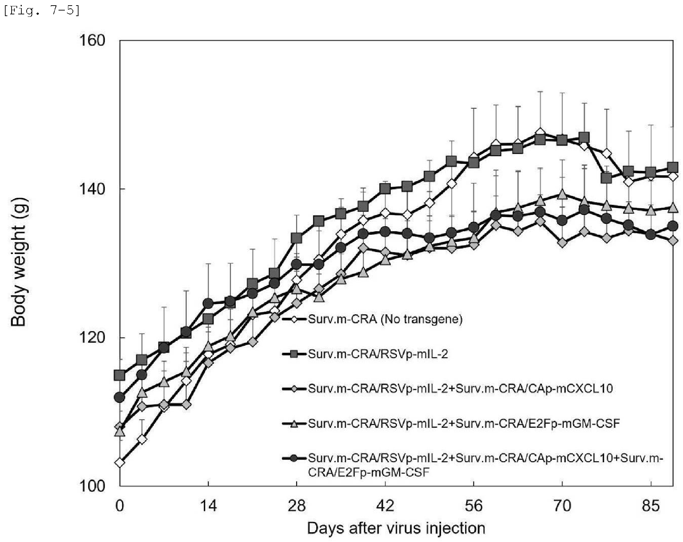

   Fig. 7-5 is a figure showing time-course body weight changes of mice during treatment in Example 7.
[Fig. 8]

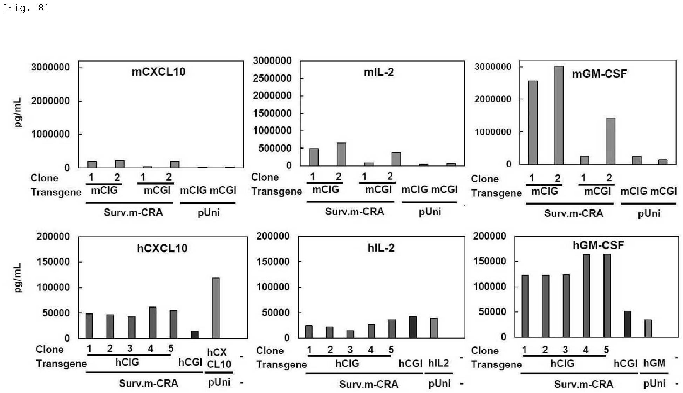

   Fig. 8 is a figure showing cytokine expression levels in cells infected with Surv.m-CRA-2 carrying three types of cytokine genes.
[Fig. 9-1]

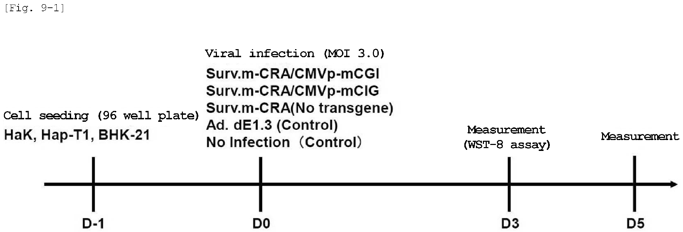

   Fig. 9-1 is a figure showing an outline of the experimental protocol in Example 10.
[Fig. 9-2]

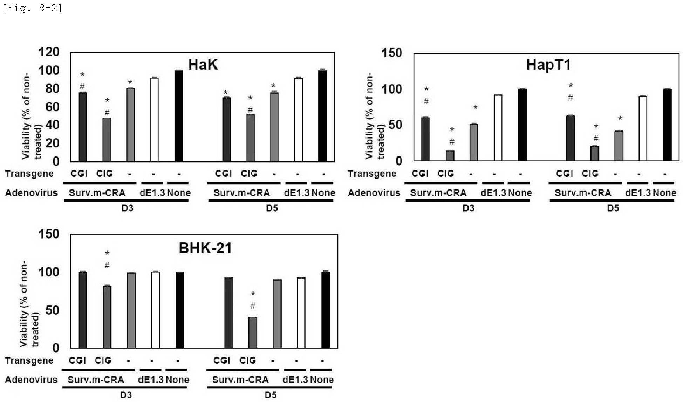

   Fig. 9-2 is a figure showing cytotoxic effects after *in vitro* infection with Surv.m-CRA-2 carrying three types of cytokine genes (n=5). *P<0.05 (vs. Ad.dE1.3); #P<0.05 (vs. Surv.m-CRA (No transgene)).
[Fig. 10-1]

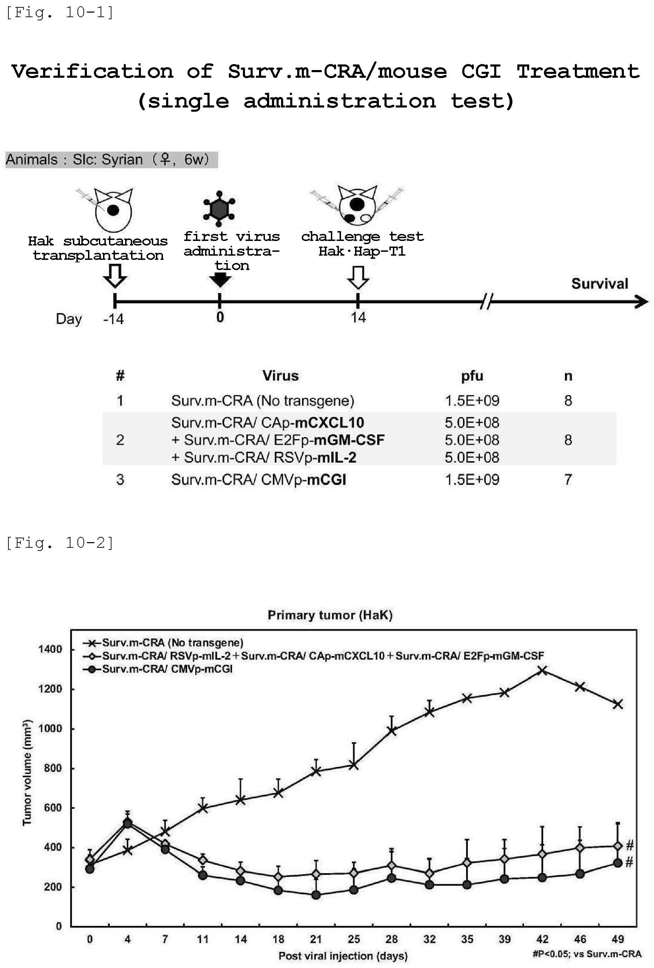

   Fig. 10-1 is a figure showing an outline of the experimental protocol in Example 11.
[Fig. 10-2]
   Fig. 10-2 is a figure showing treatment effects on primary tumors by combined use of three types of Surv.m-CRA-2 and by Surv.m-CRA-2 carrying three types of cytokine genes.
[Fig. 10-3]

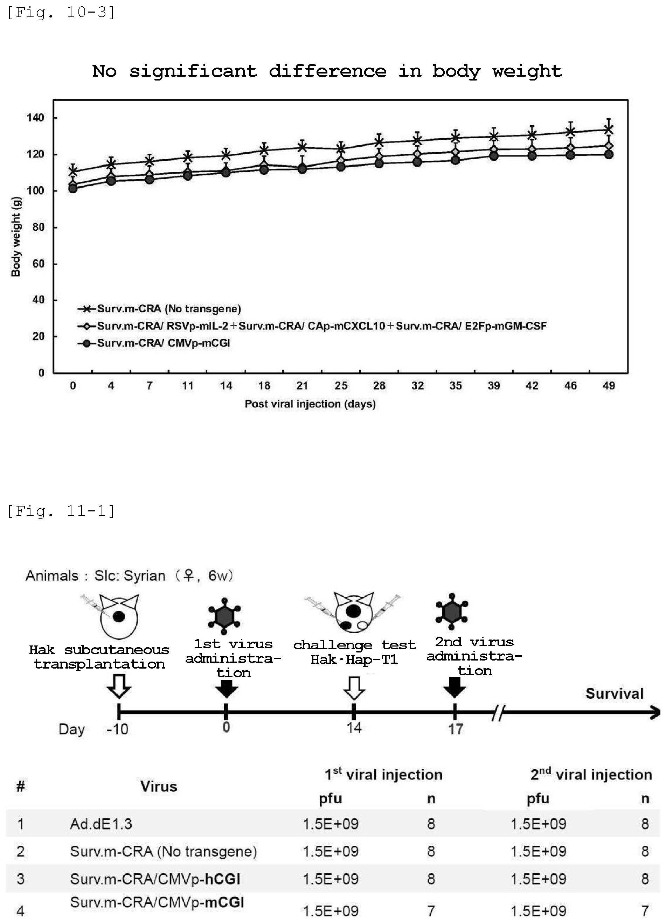

   Fig. 10-3 is a figure showing that the combined use of three types of Surv.m-CRA-2 and administration of Surv.m-CRA-2 carrying three types of cytokine genes do not affect recipient body weight.
[Fig. 11-1]
   Fig. 11-1 is a figure showing an outline of the experimental protocol in Example 12.
[Fig. 11-2]

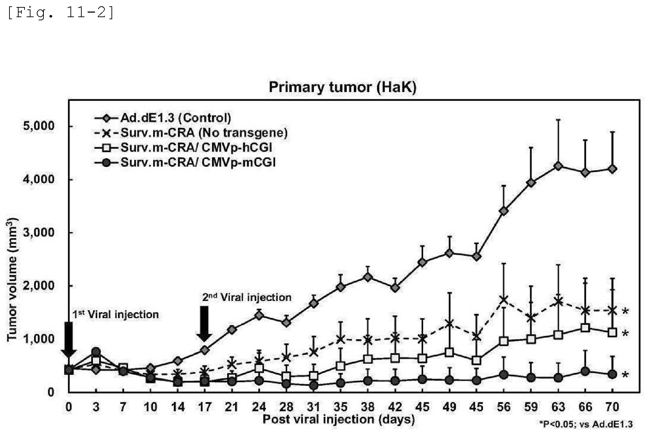

   Fig. 11-2 is a figure showing treatment effects on primary tumors by two types of Surv.m-CRA-2 carrying three types of mouse-derived and human-derived cytokine genes and Surv.m-CRA carrying no therapeutic gene.
[Fig. 11-3]

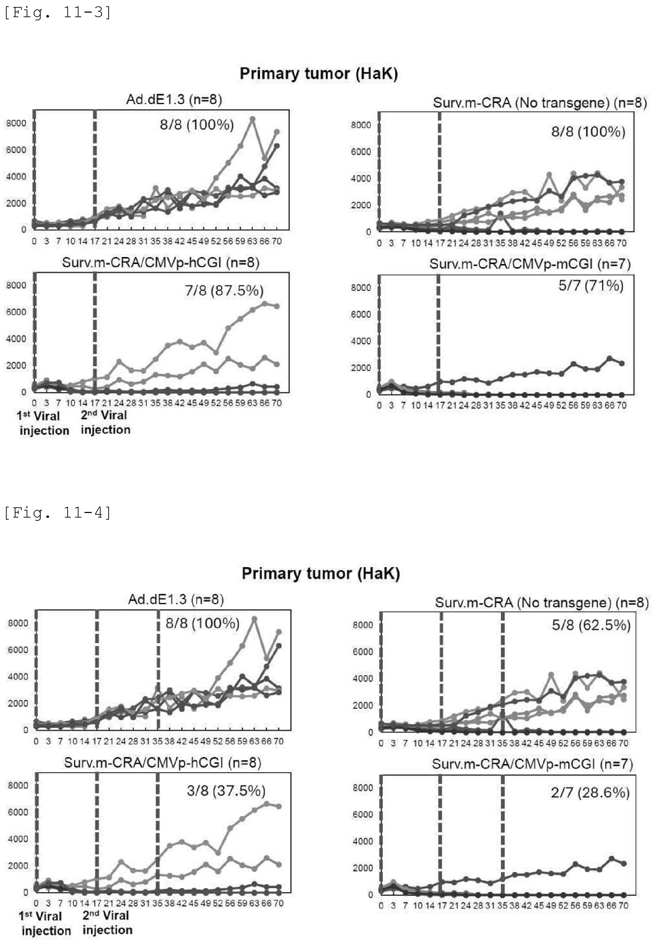

   Fig. 11-3 is a figure showing treatment effects on primary tumors on an individual basis by two types of Surv.m-CRA-2 carrying three types of mouse-derived and human-derived cytokine genes and Surv.m-CRA carrying no therapeutic gene. The tumor formation rate at 17 days after the first virus administration is shown in the upper right of the graph.
[Fig. 11-4]
   Fig. 11-4 is a figure showing treatment effects on primary tumors on an individual basis by two administrations of two types of Surv.m-CRA-2 carrying three types of mouse-derived and human-derived cytokine genes and Surv.m-CRA carrying no therapeutic gene. The tumor formation rate at 17 days after the second virus administration (35 days after the first administration) is shown in the upper right of the graph.

### [Description of Embodiments]

The present invention provides an oncolytic virus (hereinafter also referred to as "the oncolytic virus of the present invention") carrying the CXCL10 gene as an immunostimulatory gene. The oncolytic virus of the present invention is surprisingly characterized by a higher tumor growth suppressive effect, when used alone, at least in the primary tumor, compared with a control oncolytic virus that does not carry the CXCL10 gene. In the present specification, "high treatment effect" such as tumor growth suppression is used to also include cases where no significant difference is found but the treatment effect tends to increase, though it is desirable for the effect to be statistically significantly higher (e.g., p<0.05) compared with the comparison target.

CXCL10 is a chemokine that induces the infiltration and migration of immune cells such as CXCR3-positive NK cells and T cells into tumors. However, previous studies have reported that in parvoviruses carrying CXCL10, the expression of the NS1 protein involved in viral replication is suppressed for some reason, resulting in an attenuated treatment effect (Dinsart et al., Hum Gene Ther, 2017, 28(3): 295-306). Furthermore, adenoviruses carrying CXCL10 increased the infiltration of immune cells into tumors, but they could not suppress tumor growth when used alone (Li et al., Oncoimmunology, 2022, 11(1): 2118210). Therefore, that the oncolytic virus of the present invention can, when used alone, suppress tumor growth even more powerfully than oncolytic viruses that do not carry therapeutic genes is an exceptionally remarkable effect that even those of ordinary skill in the art could not have anticipated.

The oncolytic virus of the present invention contains nucleic acid encoding CXCL10 under the control of a promoter that functions in cancer cells. Here, "functional in cancer cells" means having transcriptional activity that induces CXCL10 expression at least within cancer cells, and does not question whether or not it has transcriptional activity in cells other than cancer cells. Examples of promoters that function in cancer cells include ubiquitous promoters, cancer cell-specific promoters, organ-specific promoters of the organ from which the cancer cells are derived, and the like. Furthermore, in the present specification, "cancer cell-specific" and "organ specific" are not limited to cases where there is absolutely no transcriptional activity in normal cells or other organs, but also include cases where gene expression is driven in normal cells or cells of other organs to a therapeutically acceptable extent.

As ubiquitous promoter, for example, cytomegalovirus (CMV)-derived promoter (e.g., CMV immediate-early promoter; also referred to simply as "CMV promoter" in the present specification), Rous sarcoma virus (RSV)-derived promoter (e.g., RSV LTR; also referred to simply as "RSV promoter" in the present specification), chicken β-actin gene promoter with added cytomegalovirus immediate-early gene enhancer (also referred to as "CA promoter" in the present specification), human immunodeficiency virus (HIV)-derived promoter (e.g., HIV LTR), mouse mammary cancer virus (MMTV)-derived promoter (e.g., MMTV LTR), Moloney murine leukemia virus (MoMLV)-derived promoter (e.g., MoMLV LTR), Herpes simplex virus (HSV)-derived promoter (e.g., HSV thymidine kinase (TK) promoter), SV40-derived promoter (e.g., SV40 early promoter), Epstein-Barr virus (EBV)-derived promoter, Adeno-associated virus (AAV)-derived promoter (e.g., AAV p5 promoter), Adenovirus (AdV)-derived promoter (e.g., Ad2 or Ad5 major late promoter), β-actin gene promoter, PGK gene promoter, transferrin gene promoter, or the like can be used.

Examples of cancer cell-specific promoter include CEA (carcinoembryonic antigen) promoter (Mol. Cell. Biol., 10(6), 2738-2748, 1990), E2F promoter (Neuman, E. et al., Mol. Cell. Biol., 14(10), 6607-6615, 1994), OC (osteocalcin) promoter (Morrison, N.A. et al., Science, 246, 1158-1161, 1989), FLK-1 promoter specific to malignant melanoma and fibrosarcoma (Xie, B. et al., Br. J. Cancer, 81, 1335-1343, 1999), VEGF promoter specific to lung cancer (Koshikawa, N. et al., Cancer Res., 60, 2936-2941, 2000), c-Myc promoter specific to small cell lung cancer (Kumagai, T. et al., Cancer Res., 354-358, 1996), SLPI promoter specific to lung cancer and ovarian cancer (Garver, R.I. et al., Gene Ther., 1, 46-50, 1994), PSA promoter specific to prostate cancer (Latham, J.P. et al., Cancer Res., 60, 334-342, 2000), Tyrosinase promoter specific to malignant melanoma (Vile, R.G. et al., Cancer Res., 53, 962-967, 1993), AP-2 promoter specific to breast cancer (Pandha, H.S. et al., J. Clin. Oncol., 17, 2180-2189, 1999), telomerase reverse transcriptase (TERT) promoter specific to many cancers including brain tumors (Takakura, M. et al., Cancer Res., 59, 551-557, 1999), hypoxia-responsive region (HRE) promoter specific to various cancers, Grp78 promoter, L-plastin promoter, hexokinase II promoter, survivin promoter, Aurora kinase A promoter, Aurora kinase B promoter, and the like.

Organ-specific promoters of the organ from which the cancer cells are derived are appropriately selected according to the organ from which the cancer to be treated is derived. Examples include promoters for albumin and α-fetoprotein that are specific to the liver and the like, promoter for prostate-specific antigen (PSA) that is specific to the prostate, promoter for mitochondrial creatine kinase (MCK) that is specific to various organs such as muscle and brain, and promoters for myelin basic protein (MB), glial fibrillary acidic protein (GFAP), and neuron-specific enolase (NSE) that are specific to the nervous system including the brain.

In another embodiment, the oncolytic virus of the present invention may contain a nucleic acid encoding CXCL10 under the control of an inducible promoter. As an inducible promoter, for example, metallothionein-1 gene promoter or the like can be used. When the metallothionein-1 gene promoter is used, CXCL10 can be expressed in cancer cells by administering an inducing substance such as a heavy metal (e.g., gold, zinc, cadmium, etc.), steroid (e.g., dexamethasone, etc.), an alkylating agent, a chelating agent, or a cytokine to the tumor site at a desired time.

As a promoter to control CXCL10 expression, it is desirable to choose one that can provide a suitable gene expression level from the perspective of both treatment effect and safety. Cytokine genes exhibit high physiological activity even at low expression levels, and overexpression thereof has the risk of causing undesirable side reaction such as cytokine storm. In addition, unlike non-replicating viruses, oncolytic viruses proliferate in large quantities in cancer cells. Therefore, therapeutic genes carried on viral vectors are expressed in large quantities within cancer cells and can be released at high levels from destroyed cancer cells. It is thus desirable to select a promoter that provides an expression level of CXCL10 that does not cause undesirable side reaction or causes side reaction of a tolerable level in the subject of administration, as long as suppression of tumor growth in cancer primary lesion, and induction of cancer-specific systemic antitumor immunity sufficient to suppress cancer in invasive and distant metastatic sites can be achieved. The selection of such a preferable promoter, when using adenovirus as an oncolytic virus, can be carried out, for example, by constructing, using the m-CRA technology developed by the present inventors, an m-CRA panel in which nucleic acids encoding CXCL10 are linked downstream of various candidate promoters, then administering same to, for example, cancer cell lines or a hamster tumor-bearing model system established by the present inventors that allows adenovirus proliferation, and analyzing the expression levels, treatment effects, and side reactions thereof.

In one preferred embodiment, a nucleic acid encoding CXCL10 under the control of CA promoter, CMV promoter, RSV promoter, or E2F promoter can be configured. As shown in the Examples described later, these promoters, in this order, exhibit high transcriptional activity in cancer cells, but any of the promoters can kill various cancer cells *in vitro* almost equivalently. Also in a hamster model, use of the CMV promoter having strong transcriptional activity results in superior therapeutic effects without causing serious side effects, compared with a control oncolytic virus without therapeutic genes.

As the CA promoter, CMV promoter, RSV promoter, and E2F promoter to be used in the present invention, nucleotide sequences represented by SEQ ID NOs: 1, 2, 3, and 4, respectively, or nucleic acids containing nucleotide sequences that hybridize to a complementary strand sequence of each of the nucleotide sequences under stringent conditions, and have cancer cell-specific transcriptional activity equivalent to that of the promoter composed of each of the nucleotide sequences can be mentioned. As such nucleic acid, a nucleic acid containing a nucleotide sequence having an identity of about 80% or more, preferably about 90% or more, more preferably about 95% or more, particularly preferably about 97% or more, most preferably about 98% or more, with the nucleotide sequence shown by each SEQ ID NO, and the like can be mentioned. The identity of the nucleotide sequence in the present specification can be, for example, calculated using homology calculation algorithm NCBI BLAST (National Center for Biotechnology Information Basic Local Alignment Search Tool) under the following conditions (expectancy=10; allowing gap; filtering=ON; match score=1; mismatch score=-3).

A promoter that controls expression of CXCL10 can be prepared by cloning genomic DNA containing the promoter region from genomic DNA extracted from human or other mammalian cells or tissues using, as a probe, a nucleic acid consisting of a known promoter sequence (e.g., a nucleotide sequence represented by any of SEQ ID NOs: 1-4), digesting the cloned genomic DNA with a DNA-cleaving enzyme, such as an appropriate restriction enzyme, to generate DNA fragments containing a desired partial promoter sequence, separating same by gel electrophoresis, recovering the desired band, and purifying the DNA. Alternatively, the desired partial promoter sequence can also be amplified and isolated by PCR using a primer synthesized based on a known promoter sequence, and a crude extract of the above-mentioned cells or genomic DNA isolated therefrom as a template.

In addition, a promoter that controls expression of CXCL10 can also be obtained by chemically synthesizing nucleic acid containing all or part of a nucleotide sequence based on a known promoter sequence (e.g., a nucleotide sequence represented by any of SEQ ID NOs: 1-4) using a commercially available automated DNA/RNA synthesizer.

As the "nucleic acid encoding CXCL10" used in the present invention, a nucleic acid containing the nucleotide sequence shown by SEQ ID NO:5 (corresponding to nucleotide sequence (CDS) from positions 67 to 360 of human CXCL10 mRNA sequence registered in GenBank under Accession Number: NM_001565), or a nucleotide sequence that hybridizes to a complementary strand sequence thereof under stringent conditions, and encoding a protein having activity (e.g., activity that induces infiltration and migration of immune cells into tumors) equivalent to that of CXCL10 can be mentioned. Examples of the nucleic acid that hybridizes to the complementary strand sequence of the nucleotide sequence shown by SEQ ID NO:5 under stringent conditions include a nucleic acid containing a nucleotide sequence showing an identity of about 60% or more, preferably about 70% or more, more preferably about 80% or more, and particularly preferably about 90% or more, most preferably about 95% or more, with the nucleotide sequence shown by SEQ ID NO:5, and the like. The nucleic acid encodes an amino acid sequence showing an identity of about 90% or more, preferably about 95% or more, further preferably about 97% or more, and particularly preferably about 98% or more, with the amino acid sequence shown by SEQ ID NO:6, such that a protein containing the amino acid sequence has substantially the same activity (e.g., activity that induces infiltration and migration of immune cells into tumors) as a protein containing the amino acid sequence shown by SEQ ID NO:6.

The nucleic acid encoding CXCL10 may be an ortholog (e.g., nucleic acid encoding mouse CXCL10 consists of the nucleotide sequence shown by SEQ ID NO:7 (corresponding to the nucleotide sequence (CDS) from position 76 to 369 of the mouse CXCL10 mRNA sequence registered in GenBank as accession number: NM_021274)), in non-human mammals, of the nucleic acid consisting of the nucleotide sequence shown by SEQ ID NO:5. For example, it is desirable to use a nucleic acid encoding CXCL10 derived from the mammal to which the virus is administered. The animal to be the subject of administration of the oncolytic virus of the present invention is not particularly limited as long as it has cancer, and includes human, mouse, rat, hamster, rabbit, dog, monkey, and the like, preferably human. Therefore, in a preferred embodiment, the nucleic acid encoding CXCL10 is a nucleic acid encoding human CXCL10 (i.e., a protein consisting of the amino acid sequence shown by SEQ ID NO:6).

A nucleic acid encoding CXCL10 can be cloned by, for example, amplifying the nucleic acid by a PCR method using synthetic DNA primers having a portion of the nucleotide sequence of the CDS region of the CXCL10 gene, or by hybridizing DNA incorporated into an appropriate expression vector with a labeled DNA fragment or labeled synthetic DNA comprising the nucleotide sequence of the CDS region of the CXCL10 gene. Hybridization can be conducted according to, for example, a method described in Molecular Cloning, 2nd edition (J. Sambrook et al., Cold Spring Harbor Lab. Press, 1989) and the like.

The nucleotide sequence of DNA can be converted according to a method known per se, such as the ODA-LA PCR method, the Gapped duplex method, the Kunkel method and the like, or a method based thereon, using a publicly known kit, for example, Mutan^{TM}-super Express Km (Takara Shuzo Co., Ltd.), Mutan^{TM}-K (Takara Shuzo Co., Ltd.) and the like.

The cloned DNA can be used as is, or after digestion with a restriction endonuclease or addition of a linker as desired, depending on the purpose of its use. The DNA may have the translation initiation codon ATG at the 5' end thereof, and the translation stop codon TAA, TGA or TAG at the 3' end thereof. These translation initiation codon and translation stop codon can be added using an appropriate synthetic DNA adapter.

An expression vector comprising a nucleic acid encoding CXCL10 can be produced, for example, by excising a desired fragment from a nucleic acid encoding the CDS region of the CXCL10 gene and ligating the fragment downstream of a promoter in the above-mentioned expression vector. The expression vector preferably contains a transcription termination signal, i.e., a terminator region, downstream of the nucleic acid encoding CXCL10. The expression vector can further contain a selection marker gene for selection of transformed cells (genes that offer resistance against agents such as tetracycline, ampicillin, kanamycin, hygromycin, and phosphinothricin, genes that complement an auxotrophic mutation, and the like).

The oncolytic virus of the present invention is not particularly limited in type, as long as it proliferates specifically in cancer cells, kills (lyses) the cancer cells, and its daughter viruses successively reinfect nearby cancer cells to exert a tumor-suppressive effect. Cancer selectivity may be conferred by any mechanism, but it must be capable of carrying at least a CXCL10 expression cassette as an immunostimulatory gene. Examples of oncolytic viruses that have been marketed or progressed to clinical trials include adenovirus, herpes simplex virus (HSV), vaccinia virus, measles virus, reovirus, Newcastle disease virus, coxsackievirus, and parvovirus. In the case of HSV, deleting genes involved in DNA synthesis, such as ribonucleotide reductase and thymidine kinase eliminates pathogenicity in normal cells, allowing the virus to proliferate only in cancer cells where cell proliferation is activated. In addition, the γ34.5 gene product prevents PKR-mediated inhibition of viral replication, and deleting this gene reduces pathogenicity in normal cells, allowing viral replication only in cancer cells expressing Ras, which inhibits PKR. In the case of vaccinia virus, for example, inactivating the thymidine kinase gene allows the virus to proliferate in a thymidine kinase-dependent manner in host cancer cells. In the case of measles virus, viral replication becomes possible in various cancer cells that highly express CD46, which is the receptor used by the measles vaccine strain.

In preferred embodiments, the oncolytic virus of the present invention has a promoter of a nucleic acid encoding at least one factor essential for viral replication or assembly replaced with a cancer cell-specific promoter or an organ-specific promoter of the organ from which the cancer cells are derived.

The "factor essential for viral replication or assembly" means any protein essential for viral replication or assembly, such as viral proteins required for the transcription of various viral genes (for example, in adenovirus, transcription of the early genes E1A, E1B, E2, and E4 is required before transcription of viral structural proteins, and in particular, E1A is the first protein to be transcribed and translated after infection, and the transcription of this E1A protein is essential for the initiation of transcription of various viral proteins that occur subsequently), or viral structural proteins (for example, in adenovirus, late gene products such as L1, L2, L3, L4, L5, etc.). Such factors vary depending on the type of virus used. For example, in the case of adenovirus, they include E1A, E1B, E2, and E4, preferably E1A and E1B, more preferably E1A. In the case of adeno-associated viruses, they include Rep78 and Rep68 under the control of the p5 promoter, and Rep52 and Rep40 under the control of the p19 promoter. In the case of herpes simplex virus, they include early gene products such as ICP0, ICP4, ICP22, and ICP27, and thymidine kinase. In the case of Sendai virus, they include N protein, P protein, and L protein.

The factor essential for viral replication is essential for inducing the cellular environment necessary for viral proliferation in normal cells, but may also be those with deleted regions that are not necessary for viral proliferation in cancer cells. For example, in normal cells, inactivation of Rb and p53 is necessary to drive the cell cycle for viral replication. However, in cancer cells, the cell cycle is already in progress. Therefore, in the case of adenovirus, for example, the Rb-binding region of E1A and the p53-binding region of E1B are not necessary for viral replication in cancer cells. Accordingly, the conditionally replicating adenovirus (CRA) of the present invention enables cancer cell-specific viral replication by deleting the E1A24KDa region (E1AΔ24), the E1B55KDa region (E1BΔ55K), or the E1B19KDa region (E1BΔ19).

At least one endogenous promoter of a gene encoding the above-mentioned factor essential for viral replication or assembly is replaced with a cancer cell-specific promoter or an organ-specific promoter of the organ from which the cancer cells are derived. Here, as the "cancer cell-specific promoter" and the "organ-specific promoter of the organ from which the cancer cells are derived", the promoters respectively exemplified above as those that control the expression of CXCL10 can be preferably used similarly. Preferably, the cancer cell-specific promoter can be survivin promoter, Aurora kinase A, or Aurora kinase B promoter, more preferably survivin promoter.

The promoters of the mouse and human Survivin gene have been isolated, and the sequence information thereof has been disclosed (see, for example, Li, F. and Altieri, D.C., Cancer Res., 59: 3143-3151, 1999; Li, F. and Altieri, D.C., Biochem. J., 344: 305-311, 1999).

The survivin promoter used in the oncolytic virus of the present invention is the promoter of the human Survivin gene or its ortholog gene in other mammals (e.g., monkeys, cattle, horses, pigs, dogs, cats, sheep, goats, rabbits, mice, rats, etc.), preferably the promoter of the human or mouse-derived Survivin gene (including the nucleotide sequences represented by SEQ ID NOs. 9 and 10, or partial sequences thereof). While it is preferable to use the same type of survivin promoter as the target mammal, heterologous promoters may also be used as long as they exhibit sufficient promoter activity to provide sufficient infection efficiency and killing effect on cancer cells. For example, an oncolytic virus containing the mouse survivin gene promoter can be used as a therapeutic vector for human cancer.

The Aurora kinase promoter used in the oncolytic virus of the present invention is not particularly limited as long as it is a promoter derived from a gene belonging to the Aurora kinase family. Examples include mammalian (e.g., human, monkey, cattle, horse, pig, dog, cat, sheep, goat, rabbit, mouse, rat, etc.) orthologues of the Drosophila Aurora-A, -B, and C genes. Preferably, a promoter of the Aurora kinase A gene or Aurora kinase B gene derived from a human or other mammal is used, more preferably a human Aurora kinase A or human Aurora kinase B promoter. Depending on the mammal as the treatment target, it is preferable to use Aurora kinase promoter derived from the same species, but heterologous promoters may also be used as long as they can exhibit sufficient promoter activity to provide sufficient infection efficiency and killing effect to cancer cells.

The nucleotide sequence length of the survivin promoter and the Aurora kinase promoter is not particularly limited as long as it is specific to target cancer cells and can activate the transcription of genes linked downstream to the extent that it can exert sufficient therapeutic activity against cancer. For example, sequences with the lengths described in WO 2019/093435 can be used. More specifically, for example, in the case of the mouse survivin promoter, the desired specificity and transcriptional activity can be obtained if it contains the nucleotide sequence from position -173 to -19 with the translation start site as +1 (nucleotide sequence from positions 1124 to 1278 in the nucleotide sequence shown in SEQ ID NO: 10), and in the case of the human survivin promoter, if it contains the nucleotide sequence from position -173 to -1 with the translation start site as +1 (nucleotide sequence from positions 1296 to 1468 in the nucleotide sequence shown in SEQ ID NO: 9). Therefore, preferably, the survivin promoter used in the present invention comprises at least the partial nucleotide sequence from position 1124 to 1278 in the nucleotide sequence shown in SEQ ID NO: 10, or at least the partial nucleotide sequence from position 1296 to 1468 in the nucleotide sequence shown in SEQ ID NO: 9. In one preferred embodiment, the survivin promoter substantially consists of the said partial nucleotide sequence. For preferred nucleotide sequences of the Aurora kinase promoter, WO 2019/093435 can be referred to.

A conditionally replicating virus (CRV) dependent on a cancer cell-specific promoter or an organ-specific promoter of the organ from which the cancer cells are derived cannot replicate in an environment where the promoter is not activated (e.g., normal cells), and therefore the cells are not damaged. On the other hand, when cancer cell-specific or organ-specific promoter-dependent CRV enters an environment where the promoter is activated (e.g., cancer cells), the virus replicates there, and the cells are damaged by the cytotoxicity of the viral proteins. Viruses released from lysed cells successively infect cells into which the vector has not been introduced, and the same steps are repeated. Thus, theoretically, the CRV can eventually be introduced into all cancer cells within the lesion.

If at least one nucleic acid encoding a factor essential for viral replication or assembly is under the control of a cancer cell-specific or organ-specific promoter, viral replication or assembly is limited to the environment in which the promoter is activated. Therefore, other nucleic acids encoding factors essential for viral replication or assembly may be under the control of any exogenous promoter different from said promoter. For example, the ubiquitous promoter, cancer cell-specific promoter, organ-specific promoter of the organ from which the cancer cells are derived, and inducible promoter exemplified as promoters controlling the expression of CXCL10 can be similarly used preferably.

Also, when two or more nucleic acids encoding factors essential for viral replication or assembly are under the control of the same cancer cell-specific or organ-specific promoter, they may be polycistronically configured under the control of a single promoter, or they may be monocistronically controlled by separate promoters.

Alternatively, as nucleic acids encoding factors essential for viral replication controlled by promoters other than cancer cell-specific or organ-specific promoters, nucleic acids encoding the above-mentioned mutant viral proteins (e.g., E1AΔ24, E1BΔ55K) that are essential for inducing the cellular environment necessary for viral proliferation in normal cells but lack regions not necessary for viral proliferation in target cancer cells can also be used.

In one preferred embodiment, in the CRA of the present invention, the promoter of a nucleic acid encoding E1A is replaced with a survivin promoter, and the promoter of a nucleic acid encoding E1BΔ55K is replaced with an exogenous promoter selected from a ubiquitous promoter, a cancer cell-specific promoter, and an organ-specific promoter of the organ from which the cancer cells are derived. In a particularly preferred embodiment, the exogenous promoter is a CMV promoter.

In one preferred embodiment of the present invention, a multifactor cancer-specific growth-control type recombinant adenovirus system (m-CRA; JP 2005-046101 A and WO 2005/012536) developed by the present inventors is used. An example of a plasmid vector preferably used for constructing m-CRA is illustrated in the above-mentioned Patent Literature. In the Figure, the survivin promoter, etc., can be used as promoter A and/or promoter B of plasmid vector P1, and any promoter that controls expression of the aforementioned CXCL10 can be used as promoter C of plasmid vector P2.

In the specific embodiments shown in the Examples described below, plasmid vector P1 is provided, comprising an E1A gene (which may lack a 24kDa region) functionally linked to the survivin promoter and an E1B gene (which may lack 19kDa or 55kDa region) functionally linked to a ubiquitous promoter (e.g., CMV promoter); plasmid vector P2 is provided, containing nucleic acid encoding CXCL10 functionally linked to a ubiquitous promoter (e.g., CA promoter, CMV promoter, RSV promoter) or a cancer cell-specific promoter (e.g., E2F promoter); and backbone plasmid P3 is provided, containing an adenovirus genome (which may have target cell-specific mutations within the fiber gene) deleting the E1 region. These three types of plasmids are appropriately combined, and plasmid fusion is performed using the Cre recombinase loxP system. By selecting the target plasmid using the drug resistance genes and ori contained in each plasmid, cancer cell-specific proliferation type adenovirus (CRA) vector plasmids are constructed, carrying a survivin promoter-E1A expression cassette, a ubiquitous promoter-E1B expression cassette, and a ubiquitous or cancer cell-specific promoter-CXCL10 expression cassette. Subsequently, the CRA vector can be produced by transfecting a cell line complementary to E1A (e.g., 293 cells) using this vector.

The present invention also provides an oncolytic virus in which the CXCL10 gene is combined with the IL-2 and/or GM-CSF gene as an immunostimulatory gene. Here, the nucleic acid encoding IL-2 and/or the nucleic acid encoding GM-CSF may be contained together with the nucleic acid encoding CXCL10 in a single oncolytic virus, or they may be contained in an oncolytic virus separate from the nucleic acid encoding CXCL10.

When the nucleic acid encoding IL-2 and the nucleic acid encoding GM-CSF are each contained in separate oncolytic viruses, the present invention provides a combination of the aforementioned oncolytic virus of the present invention, and
(a) an oncolytic virus containing a nucleic acid encoding IL-2 under the control of a promoter functional in cancer cells, and/or
(b) an oncolytic virus containing a nucleic acid encoding GM-CSF under the control of a promoter functional in cancer cells.

The promoters functional in cancer cells in the oncolytic viruses of the above-mentioned (a) and (b) may, independently, be a ubiquitous promoter, a cancer cell-specific promoter, or an organ-specific promoter of the organ from which the cancer cells are derived. As these promoters, the ubiquitous promoter, cancer cell-specific promoter, and organ-specific promoter of the organ from which the cancer cells are derived, as exemplified as promoters controlling CXCL10 expression for the oncolytic viruses of the present invention, can be preferably used similarly. The promoters functional in cancer cells in the oncolytic viruses of (a) and/or (b) may be the same as or different from the promoters controlling CXCL10 expression in the oncolytic virus of the present invention. In one preferred embodiment, the promoter controlling IL-2 expression and GM-CSF expression are each independently a CA promoter, a CMV promoter, an RSV promoter, or an E2F promoter.

In a preferred embodiment, as the oncolytic viruses of (a) and (b), oncolytic viruses may be used that have the same configuration as the aforementioned oncolytic virus containing a nucleic acid encoding CXCL10, except that the nucleic acid encoding CXCL10 is replaced with a nucleic acid encoding IL-2 or GM-CSF. Therefore, in one particularly preferred embodiment, the oncolytic viruses of (a) and (b) are CRAs, similar to the aforementioned oncolytic virus of the present invention, and the structure of the replication control region of each oncolytic virus is also identical to that of the oncolytic virus of the present invention. In particular, in the oncolytic virus of the present invention and the oncolytic viruses of the above-mentioned (a) and (b), it is desirable that the promoter of the nucleic acid encoding E1A is replaced with a survivin promoter, and further, the promoter of the nucleic acid encoding E1BΔ55K is replaced with an exogenous promoter selected from a ubiquitous promoter, a cancer cell-specific promoter, and an organ-specific promoter of the organ from which the cancer cells are derived, preferably a CMV promoter.

As the "nucleic acid encoding IL-2" used in the present invention, a nucleic acid containing the nucleotide sequence shown by SEQ ID NO:11 (corresponding to nucleotide sequence (CDS) from positions 286 to 744 of human IL-2 mRNA sequence registered in GenBank under Accession Number: NM_000586), or a nucleotide sequence that hybridizes to a complementary strand sequence thereof under stringent conditions, and encoding a protein having activity (e.g., T cell stimulation activity) equivalent to that of IL-2 can be mentioned. Examples of the nucleic acid that hybridizes to the complementary strand sequence of the nucleotide sequence shown by SEQ ID NO:11 under stringent conditions include a nucleic acid containing a nucleotide sequence showing an identity of about 60% or more, preferably about 70% or more, more preferably about 80% or more, and particularly preferably about 90% or more, most preferably about 95% or more, with the nucleotide sequence shown by SEQ ID NO:11, and the like. The nucleic acid encodes an amino acid sequence showing an identity of about 90% or more, preferably about 95% or more, further preferably about 97% or more, and particularly preferably about 98% or more, with the amino acid sequence shown by SEQ ID NO:12, such that a protein containing the amino acid sequence has substantially the same activity (e.g., T cell stimulation activity) as a protein containing the amino acid sequence shown by SEQ ID NO:12.

The nucleic acid encoding IL-2 may be an ortholog (e.g., nucleic acid encoding mouse IL-2 consists of the nucleotide sequence shown by SEQ ID NO:13 (corresponding to the nucleotide sequence (CDS) from position 49 to 555 of the mouse IL-2 mRNA sequence registered in GenBank as accession number: NM_008366)), in non-human mammals, of the nucleic acid consisting of the nucleotide sequence shown by SEQ ID NO:11. For example, it is desirable to use a nucleic acid encoding IL-2 derived from the mammal that is the administration subject. The animal to be the subject of administration of the oncolytic virus of the present invention is not particularly limited as long as it has cancer, and includes human, mouse, rat, hamster, rabbit, dog, monkey, and the like, preferably human. Therefore, in a preferred embodiment, the nucleic acid encoding IL-2 is a nucleic acid encoding human IL-2 (i.e., a protein consisting of the amino acid sequence shown by SEQ ID NO:12).

As the "nucleic acid encoding GM-CSF" used in the present invention, a nucleic acid containing the nucleotide sequence shown by SEQ ID NO:15 (corresponding to nucleotide sequence (CDS) from positions 36 to 467 of human GM-CSF mRNA sequence registered in GenBank under Accession Number: NM_000758), or a nucleotide sequence that hybridizes to a complementary strand sequence thereof under stringent conditions, and encoding a protein having activity (e.g., antigen-presenting capacity enhancing activity) equivalent to that of GM-CSF can be mentioned. Examples of the nucleic acid that hybridizes to the complementary strand sequence of the nucleotide sequence shown by SEQ ID NO:15 under stringent conditions include a nucleic acid containing a nucleotide sequence showing an identity of about 60% or more, preferably about 70% or more, more preferably about 80% or more, and particularly preferably about 90% or more, most preferably about 95% or more, with the nucleotide sequence shown by SEQ ID NO:15, and the like. The nucleic acid encodes an amino acid sequence showing an identity of about 90% or more, preferably about 95% or more, further preferably about 97% or more, and particularly preferably about 98% or more, with the amino acid sequence shown by SEQ ID NO:16, such that a protein containing the amino acid sequence has substantially the same activity (e.g., antigen-presenting capacity enhancing activity) as a protein containing the amino acid sequence shown by SEQ ID NO:16.

The nucleic acid encoding GM-CSF may be an ortholog (e.g., nucleic acid encoding mouse GM-CSF consists of the nucleotide sequence shown by SEQ ID NO:17 (corresponding to the nucleotide sequence (CDS) from position 290 to 712 of the mouse GM-CSF mRNA sequence registered in GenBank as accession number: NM_009969)), in non-human mammals, of the nucleic acid consisting of the nucleotide sequence shown by SEQ ID NO:15. For example, it is desirable to use a nucleic acid encoding GM-CSF derived from the mammal that is the administration subject. The animal to be the subject of administration of the oncolytic virus of the present invention is not particularly limited as long as it has cancer, and includes human, mouse, rat, hamster, rabbit, dog, monkey, and the like, preferably human. Therefore, in a preferred embodiment, the nucleic acid encoding GM-CSF is a nucleic acid encoding human GM-CSF (i.e., a protein consisting of the amino acid sequence shown by SEQ ID NO:18).

A nucleic acid encoding IL-2 or GM-CSF can be cloned, for example, by amplifying same by the PCR method using a synthetic DNA primer containing a portion of the nucleotide sequence of the CDS region of IL-2 or GM-CSF gene, or by hybridizing a DNA incorporated in an appropriate expression vector to a labeled DNA fragment containing the nucleotide sequence containing the nucleotide sequence of the CDS region of the IL-2 or GM-CSF gene, or labeled synthetic DNA. Hybridization can be conducted according to, for example, a method described in Molecular Cloning, 2nd edition (mentioned above) and the like.

The nucleotide sequence of DNA can be converted according to a method known per se, such as the ODA-LA PCR method, the Gapped duplex method, the Kunkel method and the like, or a method based thereon, using a publicly known kit, for example, Mutan^{TM}-super Express Km (Takara Shuzo Co., Ltd.), Mutan^{TM}-K (Takara Shuzo Co., Ltd.) and the like.

The cloned DNA can be used as is, or after digestion with a restriction endonuclease or addition of a linker as desired, depending on the purpose of its use. The DNA may have the translation initiation codon ATG at the 5' end thereof, and the translation stop codon TAA, TGA or TAG at the 3' end thereof. These translation initiation codon and translation stop codon can be added using an appropriate synthetic DNA adapter.

An expression vector containing a nucleic acid encoding IL-2 or GM-CSF can be produced, for example, by cutting out a desired fragment from the nucleic acid encoding CDS region of the IL-2 or GM-CSF gene, and ligating the fragment downstream of a promoter in the above-mentioned expression vector. The expression vector preferably contains a transcription termination signal, i.e., terminator region, the downstream of the nucleic acid encoding IL-2 or GM-CSF. The expression vector can further contain a selection marker gene for selection of transformed cells (genes that offer resistance against agents such as tetracycline, ampicillin, kanamycin, hygromycin, and phosphinothricin, genes that complement an auxotrophic mutation, and the like).

In another preferred embodiment, the oncolytic virus of the present invention contains the nucleic acid encoding IL-2 and/or the nucleic acid encoding GM-CSF together with the nucleic acid encoding CXCL10 in a single oncolytic virus. Therefore, the present invention also provides the aforementioned oncolytic virus of the present invention containing the nucleic acid encoding IL-2 under the control of a promoter functional in cancer cells, and/or the nucleic acid encoding GM-CSF under the control of a promoter functional in cancer cells. Here, the promoter that controls CXCL10 expression, and the promoter that controls IL-2 expression and/or the promoter that controls GM-CSF expression may be single promoter or different promoters. When the promoter that controls CXCL10 expression, and the promoter that controls IL-2 expression and/or the promoter that controls GM-CSF expression are the same single promoter, the nucleic acid encoding CXCL10 and the nucleic acid encoding IL-2 and/or the nucleic acid encoding GM-CSF are linked via sequences that enable polycistronic expression (e.g., IRES sequence, 2A sequence (P2A, T2A, E2A, F2A)).

When the oncolytic virus of the present invention is an adenovirus, the size of the foreign gene that can be incorporated is limited. Therefore, particularly when incorporating the nucleic acid encoding IL-2 and the nucleic acid encoding GM-CSF in addition to the nucleic acid encoding CXCL10, it is advantageous to place them under the control of a single promoter. On the other hand, if the oncolytic virus to be used allows for the insertion of larger foreign genes, it may be preferable to place each nucleic acid under the control of a separate promoter, because highly controlled expression of each immunostimulatory gene is enabled. When each nucleic acid is placed under the control of a separate promoter, each promoter may be the same or different exogenous promoter selected from ubiquitous promoters, cancer cell-specific promoters, and organ-specific promoters of the organ from which the cancer cells are derived. Since polycistronic expression may result in differences in the expression levels of each gene, it is possible to link each nucleic acid downstream of a separate, identical promoter in order to confer equivalent expression levels to each gene.

One advantage of including the nucleic acid encoding CXCL10, the nucleic acid encoding IL-2, and/or the nucleic acid encoding GM-CSF in a single oncolytic virus is improved infection and expression efficiency, and reduced dosage. If respective genes are included in separate oncolytic viruses, there is a risk that the efficiency with which they simultaneously infect cancer cells and express the genes therein may decrease, requiring two or three times the dosage to introduce the same copy number.

As an oncolytic virus containing nucleic acid encoding CXCL10, and nucleic acid encoding IL-2 and/or nucleic acid encoding GM-CSF, in a configuration capable of polycistronic expression, an oncolytic virus in which
(i) CXCL10-IL-2;
(ii) CXCL10-GM-CSF;
(iii) IL-2-CXCL10;
(iv) GM-CSF-CXCL10;
(v) CXCL10-IL-2-GM-CSF;
(vi) CXCL10-GM-CSF-IL-2;
(vii) IL-2-CXCL10-GM-CSF;
(viii) IL-2-GM-CSF-CXCL10;
(ix) GM-CSF-CXCL10-IL-2; or
(x) GM-CSF-IL-2-CXCL10
is arranged in this order from the 5' end can be mentioned.

In one preferred embodiment, in an oncolytic virus containing nucleic acid encoding CXCL10, nucleic acid encoding IL-2, and nucleic acid encoding GM-CSF, in a configuration capable of polycistronic expression, from the 5' end, the first nucleic acid and the second nucleic acid are linked via a P2A sequence, and the second nucleic acid and the third nucleic acid are linked via a T2A sequence.

The present invention provides oncolytic virus immunotherapy (OVI) using two factors of the CXCL10 gene and the IL-2 gene or the GM-CSF gene, as immunostimulatory genes, or OVI using three factors of the CXCL10 gene, the IL-2 gene, and the GM-CSF gene. The combined use of two factors increases the treatment effect compared with the CXCL10 gene alone, but the combination of three factors can further remarkably enhance the treatment effect.

When three factors are used in combination, an embodiment in which each factor is included in a separate oncolytic virus, an embodiment in which two of the factors are included in a single oncolytic virus and the other factor in a separate oncolytic virus, and an embodiment in which all three factors are included in a single oncolytic virus are considered, and any of these embodiments may be used. Therefore, in one preferred embodiment, a combination of the oncolytic virus of the present invention containing only nucleic acid encoding CXCL10 as an immunostimulatory gene, and
(c) an oncolytic virus containing nucleic acid encoding IL-2 under the control of a promoter functional in cancer cells, and nucleic acid encoding GM-CSF under the control of a promoter functional in cancer cells
is provided.

The oncolytic virus of the above-mentioned (c) can be produced in the same manner as the oncolytic virus of the present invention containing two or more immunostimulatory genes.

The present invention also provides an oncolytic virus immunotherapy agent (OVI), i.e., a cancer therapeutic agent (hereinafter also referred to as "OVI of the present invention" or "the therapeutic agent of the present invention"), which contains any of the aforementioned oncolytic viruses of the present invention or a combination of any of the aforementioned oncolytic viruses of the present invention as an active ingredient.

Cancer to be the target for the therapeutic agent of the present invention is not particularly limited and examples include, but are not limited to, renal cell cancer, fibrosarcoma, myxosarcoma, liposarcoma, chondrosarcoma, osteosarcoma, chordoma, angiosarcoma, endothelial sarcoma, lymphangiosarcoma, endolymphatic sarcoma, synovioma, mesothelioma, Ewing's tumor, leiomyosarcoma, rhabdomyosarcoma, colon cancer, pancreatic cancer, breast cancer, ovarian cancer, prostate cancer, squamous cell carcinoma, basal cell carcinoma, adenocarcinoma, sweat gland cancer, sebaceous carcinoma, papillary carcinoma, papillary adenocarcinoma, cystadenocarcinoma, medullary carcinoma, bronchogenic carcinoma, liver cancer, bile duct cancer, choriocarcinoma, seminoma, embryonal carcinoma, Wilms tumor, cervical cancer, testicular cancer, lung cancer, small cell lung cancer, bladder cancer, epithelial cancer, glioma, astrocytoma, medulloblastoma, craniopharyngioma, ependymoma, pineal gland tumor, hemangioblastoma, acoustic neuroma, oligodendroglioma, meningioma, melanoma, neuroblastoma, retinoblastoma, leukemia, acute lymphocytic leukemia, acute myeloid leukemia, chronic leukemia, polycythemia vera, lymphoma, multiple myeloma and the like. Suitable target cancers include invasive and metastatic refractory cancers for which conventional OVI and other existing cancer treatments are ineffective.

In the therapeutic agent of the present invention, the active ingredient oncolytic virus is, where necessary, mixed with a pharmacologically acceptable carrier and formulated into various forms of preparation such as injection and the like, and used as a therapeutic agent for cancer. Here, as examples of the pharmacologically acceptable carrier, various organic or inorganic carrier substances conventionally used as pharmaceutical preparation materials can be mentioned, and these are formulated as excipients, lubricants, binders, and disintegrants in solid preparations; solvents, solubilizing agents, suspending agents, isotonizing agents, buffering agents and analgesic agents in liquid preparations; and the like. Also, as necessary, pharmaceutical preparation additives such as antiseptics, antioxidants, colorants, sweetening agents, and the like can be used.

As examples of preferred excipients, lactose, sucrose, D-mannitol, D-sorbitol, starch, pregelatinized starch, dextrin, crystalline cellulose, low-substituted hydroxypropylcellulose, sodium carboxymethylcellulose, gum arabic, pullulan, light anhydrous silicic acid, synthetic aluminum silicate, magnesium alumino metasilicate and the like can be mentioned.

As examples of preferred lubricants, magnesium stearate, calcium stearate, talc, colloid silica and the like can be mentioned.

As examples of preferred binders, pregelatinized starch, sucrose, gelatin, gum arabic, methylcellulose, carboxymethylcellulose, sodium carboxymethylcellulose, crystalline cellulose, sucrose, D-mannitol, trehalose, dextrin, pullulan, hydroxypropylcellulose, hydroxypropylmethylcellulose, polyvinylpyrrolidone and the like can be mentioned.

As examples of preferred disintegrants, lactose, sucrose, starch, carboxymethylcellulose, carboxymethylcellulose calcium, croscarmellose sodium, sodium carboxymethyl starch, light anhydrous silicic acid, low-substituted hydroxypropylcellulose and the like can be mentioned.

As examples of preferred solvents, water for injection, physiological saline, Ringer's solutions, alcohols, propylene glycol, polyethylene glycol, sesame oil, corn oil, olive oil, cottonseed oil and the like can be mentioned.

As examples of preferred solubilizing agents, polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate and the like can be mentioned.

As examples of preferred suspending agents, surfactants such as stearyl triethanolamine, sodium lauryl sulfate, lauryl aminopropionic acid, lecithin, benzalkonium chloride, benzethonium chloride and glyceryl monostearate; hydrophilic polymers such as polyvinyl alcohol, polyvinylpyrrolidone, sodium carboxymethyl cellulose, methyl cellulose, hydroxymethyl cellulose, hydroxyethyl cellulose and hydroxypropyl cellulose; polysorbates, polyoxyethylene hardened castor oil and the like can be mentioned.

As examples of preferred isotonizing agents, sodium chloride, glycerin, D-mannitol, D-sorbitol, glucose and the like can be mentioned.

As examples of preferred buffers, buffer solutions of a phosphate, an acetate, a carbonate, a citrate and the like, and the like can be mentioned.

As examples of preferred anesthetic agents, benzyl alcohol and the like can be mentioned.

As examples of preferred antiseptics, paraoxybenzoates, chlorobutanol, benzyl alcohol, phenethyl alcohol, dehydroacetic acid, sorbic acid and the like can be mentioned.

As examples of preferred antioxidants, sulfites, ascorbates and the like can be mentioned.

As examples of preferred colorants, aqueous food tar colors (e.g., food colors such as Food Red Nos. 2 and 3, Food Yellow Nos. 4 and 5, and Food Blue Nos. 1 and 2), water-insoluble lake pigments (e.g., aluminum salts of the aforementioned aqueous food tar colors and the like), natural pigments (e.g., β-carotene, chlorophyll, red iron oxide and the like) and the like can be mentioned.

As examples of preferred sweetening agents, saccharin sodium, dipotassium glycyrrhizinate, aspartame, stevia and the like can be mentioned.

Administration of the therapeutic agent of the present invention is performed by either the *ex vivo* method, in which cancer cells of the treatment target animal are taken out of the body, cultured, introduced, and then returned to the body (or transplanted), or the *in vivo* method, in which the vector is directly administered into the body of the subject of administration, where the *in vivo* method is preferred. In the case of the *ex vivo* method, the introduction of the vector into target cells can be performed by microinjection, calcium phosphate coprecipitation, PEG, electroporation, and the like. In the case of *in vivo* method, the administration of the preparation can be performed, for example, by injection, catheter, balloon catheter, local injection, or implantation of an implant incorporating the oncolytic virus of the present invention into the lesion.

The dosage of the therapeutic agent of the present invention varies depending on the type of oncolytic virus, promoter activity in target cancer cells, type of immunostimulatory gene to be combined, route of administration, severity of the disease, animal species to be the subject of administration, drug acceptance of the subject of administration, weight, age, and the like. For example, when using cancer-specific replicating adenovirus as the oncolytic virus, since safety has been confirmed in conventional cancer gene therapy clinical trials using virus particles (vp) at 1x10¹⁰ - 10¹² vp/tumor, the same amount can be used as a guideline for administration (Molecular Therapy, 18: 429-434, 2010).

The therapeutic agent of the present invention can be administered as a single dose or can be administered multiple times. The number of doses and the administration interval can be selected as appropriate.

The therapeutic agent of the present invention can be used in combination with other cancer therapeutic agents or treatment methods. Examples of other cancer therapeutic agents or treatment methods include immune checkpoint inhibitors (e.g., anti-PD-1 antibodies, anti-PD-L1 antibodies, etc.), CAR-T cell therapy, chemotherapeutic agents (e.g., 5-fluorouracil, cisplatin, etc.), radiotherapy, and the like. Immune checkpoint inhibitors are known to be less effective against cancers with little immune cell infiltration. However, since the therapeutic agent of the present invention activates immune cells and promotes their infiltration into cancer tissue, a strong synergistic effect can be expected. In addition, CAR-T cell therapy has not yet achieved sufficient treatment effects against solid tumors, and the reasons therefor include difficult infiltration of CAR-T cells into the tissue of solid tumors, the presence of cancer cells that do not express the target cancer antigen, and inhibition of the function of CAR-T cells due to the immunosuppressive environment within the cancer tissue. By administering OVI of the present invention, it is expected that the expression of cytokines and chemokines will be induced, tumor infiltration of CAR-T cells will be promoted, and the immunosuppressive environment within cancer tissue will be improved to prevent suppression of CAR-T cell function.

Other cancer therapeutic agents or treatment methods to be used in combination with the therapeutic agent of the present invention may be administered or implemented in accordance with the usage and dosage when using the therapeutic agent or treatment method alone.

The present invention is described in more detail using the following examples, but the scope of the present invention is not limited in any way by these examples.

### [Example]

### [Material and method]

### [Cell culture]

The HaK cell line (Syrian hamster renal cancer cell line)was provided by Dr. William S.M. Wold (Saint Louis University School of Medicine) [Thomas, M.A., et al., Syrian hamster as a permissive immunocompetent animal model for the study of oncolytic adenovirus vectors. Cancer Res, 2006. 66(3): p. 1270-6.]. HaP-T1 (Syrian hamster pancreatic adenocarcinoma) was obtained from Riken Cell Bank (Ibaraki, Japan), and BHK-21 (Baby hamster kidney) was obtained from JCRB Cell Bank (Osaka, Japan). HaK cells were cultured using Dulbecco's Modified Eagle Medium (Nacalai Tesque, Kyoto, Japan). HaP-T1 cells were cultured in MEM containing 1% non-essential amino acids (Sigma-Aldrich, St Louis, MO) and 1 mM sodium pyruvate (Thermo Fisher Scientific, Waltham, MA). BHK-21 cells were cultured in MEM supplemented with 1% NEAA. All media were supplemented with 10% fetal bovine serum (FBS, Biowest, Nuaille, France), 100 units/mL penicillin, and 100 µg/mL streptomycin (Thermo Fisher Scientific).

### [Animal experiment]

All animal experiments were performed in accordance with the National Institutes of Health Guidelines for the Care and Use of Laboratory Animals and were approved by the Institute of Laboratory Animal Sciences, Research Support Center, Kagoshima University.

### [Distant site challenge test]

Hamsters were challenged with a tumorigenic dose of parental cancer cells (HaK) or heterologous cancer cells (HaP-T1) 2 weeks after virus administration. Briefly, hamsters bearing subcutaneous tumors were administered Surv.m-CRA (No transgene) (n=6), Surv.m-CRA/RSVp-mIL-2 (n=6), Surv.m-CRA/RSVp-mIL-2+Surv.m-CRA/CAp-mCXCL10 (n=6), Surv.m-CRA/RSVp-mIL-2+Surv.m-CRA/E2Fp-mGM-CSF (n=6), or Surv.m-CRA/RSVp-mIL-2+Surv.m-CRA/CAp-mCXCL10+Surv.m-CRA/E2Fp-mGM-CSF (n=6) by the experimental method described below. After 14 days, HaK cells and HaP-T1 cells (1×10⁷ cells per group) were inoculated into the left and right dorsal regions, respectively, of the hamsters. The animals were further macroscopically observed for 74 days for the presence of tumor nodules after various virus treatments.

### [Statistical analysis]

Data were expressed as mean±standard error. Data were evaluated by Student's two-tailed t-test. Values of P < 0.05 were considered to indicate statistical significance.

### [Example 1] Preparation of Surv.m-CRA-2 carrying cytokine genes

The E1-deleted replication-defective adenovirus (Ad.dE1.3) was prepared according to a previously reported method [Murofushi, Y., et al., Cell cycle-specific changes in hTERT promoter activity in normal and cancerous cells in adenoviral gene therapy: a promising implication of telomerase-dependent targeted cancer gene therapy. Int J Oncol, 2006. 29(3): p. 681-8., Kamizono, J., et al., Survivin-responsive conditionally replicating adenovirus exhibits cancer-specific and efficient viral replication. Cancer Res, 2005. 65(12): p. 5284-91., Kamizono, J., et al., Survivin-responsive conditionally replicating adenovirus exhibits cancer-specific and efficient viral replication. Cancer Res, 2005. 65(12): p. 5284-91., Terazaki, Y., et al., An optimal therapeutic expression level is crucial for suicide gene therapy for hepatic metastatic cancer in mice. Hepatology, 2003. 37(1): p. 155-63., Ushikoshi, H., et al., Local overexpression of HB-EGF exacerbates remodeling following myocardial infarction by activating noncardiomyocytes. Lab Invest, 2005. 85(7): p. 862-73., Sakamoto, K., et al., Heparin-binding epidermal growth factor-like growth factor and hepatocyte growth factor inhibit cholestatic liver injury in mice through different mechanisms. Int J Mol Med, 2016. 38(6): p. 1673-1682., Khai, N.C., et al., In vivo hepatic HB-EGF gene transduction inhibits Fas-induced liver injury and induces liver regeneration in mice: a comparative study to HGF. J Hepatol, 2006. 44(6): p. 1046-54., Horikawa, Y., et al., Assessment of an altered E1B promoter on the specificity and potency of triple-regulated conditionally replicating adenoviruses: implications for the generation of ideal m-CRAs. Cancer Gene Ther, 2011. 18(10): p. 724-33.]. Ad.dE1.3 does not contain a transgene.

Surv.m-CRA containing wild-type E1A downstream of the survivin promoter, E1BΔ55K downstream of the CMV promoter, and a therapeutic gene downstream of a specific promoter was prepared using the previously reported m-CRA construction method [Watanabe, M., et al., Adenovirus Biology, Recombinant Adenovirus, and Adenovirus Usage in Gene Therapy. Viruses, 2021. 13(12)., Kamizono, J., et al., Survivin-responsive conditionally replicating adenovirus exhibits cancer-specific and efficient viral replication. Cancer Res, 2005. 65(12): p. 5284-91., Tanoue, K., et al., Survivin-responsive conditionally replicating adenovirus kills rhabdomyosarcoma stem cells more efficiently than their progeny. J Transl Med, 2014. 12: p. 27., Horikawa, Y., et al., Assessment of an altered E1B promoter on the specificity and potency of triple-regulated conditionally replicating adenoviruses: implications for the generation of ideal m-CRAs. Cancer Gene Ther, 2011. 18(10): p. 724-33.].

CRAs carrying nucleic acids encoding CXCL10, GM-CSF, and/or IL-2 as therapeutic genes were prepared. Specifically, the following 16 types of CRAs were prepared.
(1) Surv.m-CRA/E2Fp-mCXCL10
(2) Surv.m-CRA/RSVp-mCXCL10
(3) Surv.m-CRA/CMVp-mCXCL10
(4) Surv.m-CRA/CAp-mCXCL10
(5) Surv.m-CRA/E2Fp-hCXCL10
(6) Surv.m-CRA/RSVp-hCXCL10
(7) Surv.m-CRA/CMVp-hCXCL10
(8) Surv.m-CRA/CAp-hCXCL10
(9) Surv.m-CRA/E2Fp-mGM-CSF
(10) Surv.m-CRA/RSVp-mIL-2
(11) Surv.m-CRA/CMV-mCXCL10-mIL2-mGM
(12) Surv.m-CRA/CMV-mCXCL10-mGM-mIL2
(13) Surv.m-CRA/CMV-hCXCL10-hIL2-hGM
(14) Surv.m-CRA/CMV-hCXCL10-hGM-hIL2
(15) Surv.m-CRA (No transgene)
(16) Surv.m-CRA/CMVp-EGFP

E2Fp indicates the E2F-1 promoter, RSVp indicates the Rous sarcoma virus long terminal repeat (RSV) promoter, CMVp indicates the human cytomegalovirus immediate-early gene enhancer/promoter (CMV promoter), and CAp indicates the CMV enhancer and β-actin (CA) promoter. The "m" or "h" attached before each cytokine name indicates mouse-derived or human-derived, respectively. (15) Surv.m-CRA (No transgene) does not express a therapeutic gene, and (16) Surv.m-CRA/CMVp-EGFP expresses the enhanced green fluorescent protein (EGFP) gene under the CMV promoter. For reference, schematic diagrams of the gene sequences of CRAs carrying mouse and human CXCL10 alone are shown in Fig. 1-1 (mouse) and Fig. 1-2 (human), respectively. In addition, a schematic diagram of the gene sequences of CRAs carrying three types of therapeutic genes is shown in Fig. 1-3 (mouse and human). In Fig. 1-3, P2A indicates a 2A sequence derived from porcine teschovirus-1, and T2A indicates a 2A sequence derived from Thosea asigna virus.

### [Example 2] In vitro functional verification of prepared Surv.m-CRA-2

Among the various CRAs prepared in Example 1, protein expression in cells was verified with respect to CXCL10.

Specifically, various P2 plasmids expressing the CXCL10 gene were transfected into HEK293 cells seeded on 6-well plates. In addition, various Surv.m-CRAs expressing the CXCL10 gene were used to infect HaK cells, HaP-T1 cells, or BHK-21 cells at MOIs of 1, 10, and 100 (CRAs carrying the human CXCL10 gene were used only for HaK cells). All cells were cultured for 48 hr, after which the supernatants were collected and frozen at -80°C. Analysis of CXCL10 protein expression in HEK293 cells and analysis of CXCL10 protein expression in HaK cells, HaP-T1 cells, or BHK-21 cells were performed as follows. Mouse CXCL10 was measured using Mouse CXCL10 DuoSet ELISA (DY466, R&D Systems, Minneapolis, MN), and human CXCL10 was measured using Human CXCL10 Quantikine ELISA Kit (DIP100, R&D Systems), according to the manufacturer's protocol. The total protein concentration of each sample was determined by Bradford assay (Bio-Rad, Hercules, CA).

The results are shown in Fig. 2-1 (A: expression of mouse CXCL10 in HEK293 cells transfected with P2 plasmids containing a nucleic acid encoding mouse CXCL10; B: expression of human CXCL10 in HEK293 cells transfected with P2 plasmids containing a nucleic acid encoding human CXCL10), Fig. 2-2 (expression of mouse CXCL10 in HaK cells, HaP-T1 cells, or BHK-21 cells infected with CRAs carrying the mouse CXCL10 gene), and Fig. 2-3 (expression of human CXCL10 in HaK cells infected with CRAs carrying the human CXCL10 gene), respectively.

As shown in Fig. 2-1 to Fig. 2-3, mouse CXCL10 protein was not detected in cells after infection with Surv.m-CRA (No transgene) or in uninfected cells. On the other hand, when infected with Surv.m-CRA/E2Fp-mCXCL10, Surv.m-CRA/RSVp-mCXCL10, Surv.m-CRA/CMVp-mCXCL10, or Surv.m-CRA/CAp-mCXCL10, secretion of mouse CXCL10 protein increased in a virus dose-dependent manner in all cell lines, at low, mild, moderate, or high levels (about 1-fold, about 10-fold, and about 100-fold differences, respectively). Furthermore, a similar tendency was observed in each Surv.m-CRA containing the human CXCL10 gene.

### [Example 3] Examination of in vitro cytotoxic effects after viral infection (WST assay)

Using Surv.m-CRAs carrying the mouse CXCL10 gene prepared in Example 1, the *in vitro* cytotoxic effect after viral infection was examined. Specifically, the following procedure was performed. HaK cells (800 cells/well), HaP-T1 cells, or BHK-21 cells (500 cells/well) seeded in 24-well plates were infected with Surv.m-CRAs or Ad.dE1.3 at an MOI of 0.3. Cell viability was measured on days 3 and 5 after infection by a WST-8 assay using Cell Count Reagent SF^{®} (Nacalai Tesque), according to the manufacturer's protocol and previously reported methods. [Tanoue, K., et al., Survivin-responsive conditionally replicating adenovirus kills rhabdomyosarcoma stem cells more efficiently than their progeny. J Transl Med, 2014. 12: p. 27., Terazaki, Y., et al., An optimal therapeutic expression level is crucial for suicide gene therapy for hepatic metastatic cancer in mice. Hepatology, 2003. 37(1): p. 155-63., Horikawa, Y., et al., Assessment of an altered E1B promoter on the specificity and potency of triple-regulated conditionally replicating adenoviruses: implications for the generation of ideal m-CRAs. Cancer Gene Ther, 2011. 18(10): p. 724-33., Mitsui, K., et al., Conditionally replicating adenovirus prevents pluripotent stem cell-derived teratoma by specifically eliminating undifferentiated cells. Mol Ther Methods Clin Dev, 2015. 2: p. 15026., Yuge, K., et al., Adenoviral gene transduction of hepatocyte growth factor elicits inhibitory effects for hepatoma. Int J Oncol, 2005. 27(1): p. 77-85.].

Briefly, the procedure was as follows.
(1) As viruses, Ad.dE1.3, Surv.m-CRA (No transgene), Surv.m-CRA/CMV-EGFP, Surv.m-CRA/E2F-mCXCL10, Surv.m-CRA/RSV-mCXCL10, Surv.m-CRA/CMV-mCXCL10, and Surv.m-CRA/CA-mCXCL10 were used (MOI:0.3).
(2) HaK cells were seeded at 800 cells/well and HaP-T1 cells or BHK-21 cells were seeded at 500 cells/well in 24-well plates.
(3) Cultured for 24 hours.
(4) Viral infection and incubation (3-5 days).
(5) Medium was replaced with 500 µL of medium containing 50 µL of Cell Count Reagent SF.
(6) Incubation (1-4 hours).
(7) Absorbance at 450 nm was measured using a microplate reader.

The results are shown in Fig. 3-1. In addition, representative phase-contrast images of HaK cells, HaP-T1 cells, and BHK-21 cells at 3 days or 5 days after viral infection are shown in Fig. 3-2 to Fig. 3-4 at magnifications of 40× (left) and 100× (right), respectively.

As shown in Fig. 3-1, Surv.m-CRA (No transgene) induced remarkable cell death in cancer cells but did not induce cell death at all in normal cells. This indicates that Surv.m-CRA replicates strictly in hamster cancer cells, as previously reported in human cells [Watanabe, M., et al., Adenovirus Biology, Recombinant Adenovirus, and Adenovirus Usage in Gene Therapy. Viruses, 2021. 13(12)., Kamizono, J., et al., Survivin-responsive conditionally replicating adenovirus exhibits cancer-specific and efficient viral replication. Cancer Res, 2005. 65(12): p. 5284-91., Tanoue, K., et al., Survivin-responsive conditionally replicating adenovirus kills rhabdomyosarcoma stem cells more efficiently than their progeny. J Transl Med, 2014. 12: p. 27., Horikawa, Y., et al., Assessment of an altered E1B promoter on the specificity and potency of triple-regulated conditionally replicating adenoviruses: implications for the generation of ideal m-CRAs. Cancer Gene Ther, 2011. 18(10): p. 724-33., Mitsui, K., et al., Conditionally replicating adenovirus prevents pluripotent stem cell-derived teratoma by specifically eliminating undifferentiated cells. Mol Ther Methods Clin Dev, 2015. 2: p. 15026., Ide, K., et al., A Novel Construction of Lentiviral Vectors for Eliminating Tumorigenic Cells from Pluripotent Stem Cells. Stem Cells, 2018. 36(2): p. 230-239.]. Expression of transgenes including mCXCL10 and EGFP showed cytotoxic effects to various degrees. Since EGFP remains intracellularly and non-immune cells do not express the receptor for CXCL10, such transgene-dependent cytotoxic effects are presumed to be due to nonspecific effects of amplified proteins derived from the transgene within infected cells.

### [Example 4] Establishment of syngeneic Syrian hamster cancer model

In order to sufficiently examine *in vivo* treatment effects, such as virus replication-dependent cytotoxic effect and cytokine-induced immune responses, HaK cells were subcutaneously transplanted into Syrian hamsters that permit replication of human serotype 5 adenovirus, and a syngeneic hamster cancer model was established. An outline of the establishment process is shown in Fig. 4.

Since human adenovirus does not proliferate in mice but can partially proliferate in Syrian hamsters, this model is considered to be usable as a refractory cancer model in Surv.m-CRA therapy.

### [Example 5] Examination of treatment effect of CXCL10-carrying Surv.m-CRA-2

Using the Syrian hamster subcutaneous tumor model established in Example 4, the treatment effect of CXCL10-carrying Surv.m-CRA-2 was examined.

The hamster model of subcutaneous tumor was prepared by transplanting 2×10⁷ HaK cells suspended in 200 µL of DMEM containing 50% Matrigel (BD Biosciences, Franklin Lakes, NJ) into the dorsal flank region of female Syrian hamsters (Japan SLC, Shizuoka, Japan) aged 5-6 weeks. After the tumor volume reached 200-580 mm³, the hamsters were randomly divided into 5-6 groups.

In monotherapy with each Surv.m-CRA expressing CXCL10, on day 0, 100 µL of buffer containing 1.0×10⁹ pfu (plaque forming unit) (10 mmol/L Tris-HCl pH 7.4, 1 mmol/L MgCl₂, 10% glycerol, and 20 µg/mL hexadimethrine bromide) was injected intratumorally once into one hamster of each group. 1.0×10⁹ pfu of Ad.dE1.3 (n=7), Surv.m-CRA (No transgene) (n=8), Surv.m-CRA/E2Fp-hCXCL10 (n=8), Surv.m-CRA/CMVp-hCXCL10 (n=7), or Surv.m-CRA/CMVp-mCXCL10 (n=8) was administered.

Thereafter, tumor size was measured twice a week, and tumor volume was calculated according to the following formula:$Volume = long axis \times {\left(short axis\right)}^{2} \times 0.5 \left({mm}^{3}\right) .$

Body weight was monitored twice a week using a digital balance.

Histopathological analysis was performed according to previous report [Kamizono, J., et al., Survivin-responsive conditionally replicating adenovirus exhibits cancer-specific and efficient viral replication. Cancer Res, 2005. 65(12): p. 5284-91., Chen, S.H., et al., Combination gene therapy for liver metastasis of colon carcinoma in vivo. Proc Natl Acad Sci U S A, 1995. 92(7): p. 2577-81., Terazaki, Y., et al., An optimal therapeutic expression level is crucial for suicide gene therapy for hepatic metastatic cancer in mice. Hepatology, 2003. 37(1): p. 155-63., Horikawa, Y., et al., Assessment of an altered E1B promoter on the specificity and potency of triple-regulated conditionally replicating adenoviruses: implications for the generation of ideal m-CRAs. Cancer Gene Ther, 2011. 18(10): p. 724-33.].

The results are shown in Fig. 5. As shown in Fig. 5, a single intratumoral administration of Surv.m-CRA (No transgene) significantly suppressed tumor growth compared with the control replication-defective Ad.dE1.3 treatment group, although significant differences were observed only at some time points. All three types of Surv.m-CRAs expressing CXCL10 under the control of different promoters (Surv.m-CRA/E2Fp-hCXCL10, Surv.m-CRA/CMVp-hCXCL10, and Surv.m-CRA/CMVp-mCXCL10) further enhanced the tumor growth suppressive effect compared with Surv.m-CRA (No transgene). This effect was most remarkable in hamsters administered Surv.m-CRA/CMVp-hCXCL10. On the other hand, in hamsters administered Surv.m-CRA/CMVp-mCXCL10, no significant difference was observed.

### [Example 6] Examination of treatment effect of Surv.m-CRA-2 by combination of two types of cytokines

It was confirmed whether the combined use of two types of cytokines enhances the treatment effect.

On day 0, 100 µL of buffer containing 2.0×10⁹ pfu of Ad.dE1.3 (n=7), Surv.m-CRA (No transgene) (n=7), Surv.m-CRA/E2Fp-mGM-CSF (n=7), Surv.m-CRA/CAp-mCXCL10 (n=7), a combination of Surv.m-CRA/CAp-mCXCL10 and Surv.m-CRA/E2Fp-mGM-CSF (n=8), or a combination of Surv.m-CRA/CAp-mCXCL10 and Surv.m-CRA/RSVp-mIL-2 (n=8) was injected intratumorally once into one hamster of each group. The method for measuring tumor size and the like was the same as in Example 5.

The results are shown in Fig. 6. As shown in Fig. 6, monotherapy with Surv.m-CRA (No transgene) or Surv.m-CRAs expressing a transgene (CXCL10 or GM-CSF) did not significantly suppress tumor growth compared with the control group. On the other hand, when Surv.m-CRA/E2Fp-mGM-CSF or Surv.m-CRA/RSVp-mIL-2 was combined with Surv.m-CRA/CAp-mCXCL10, the tumor growth suppressive effect was dramatically enhanced compared with the control. Therefore, it was suggested that combination therapy using a combination of therapeutic genes provides a synergistic treatment effect compared with monotherapy.

### [Example 7] Examination of treatment effect of Surv.m-CRA-2 by combined use of three types of cytokines

It was confirmed whether the combination of three types of cytokines enhances the treatment effect. In this Example, the treatment effect on the primary tumor and the induction of primary tumor-specific systemic antitumor immunity by a distant site challenge test were studied. An outline of the experiment is shown in Fig. 7-1.

On day 0, 100 µL of buffer containing 1.5×10⁹ pfu of Surv.m-CRA (No transgene) (n=6), Surv.m-CRA/RSVp-mIL-2 (n=6), a combination of Surv.m-CRA/RSVp-mIL-2 and Surv.m-CRA/CAp-mCXCL10 (n=6), a combination of Surv.m-CRA/RSVp-mIL-2 and Surv.m-CRA/E2Fp-mGM-CSF (n=6), or a combination of Surv.m-CRA/RSVp-mIL-2, Surv.m-CRA/CAp-mCXCL10, and Surv.m-CRA/E2Fp-mGM-CSF (n=6) was injected intratumorally once into one hamster of each group.

The distant site challenge test was performed as follows. Two weeks after virus administration, hamsters were challenged with a tumorigenic dose of parental cancer cells (HaK) or heterologous cancer cells (HaP-T1). Specifically, hamsters bearing subcutaneous tumors were treated with Surv.m-CRA (No transgene) (n=6), Surv.m-CRA/RSVp-mIL-2 (n=6), a combination of Surv.m-CRA/RSVp-mIL-2 and Surv.m-CRA/CAp-mCXCL10 (n=6), a combination of Surv.m-CRA/RSVp-mIL-2 and Surv.m-CRA/E2Fp-mGM-CSF (n=6), or a combination of Surv.m-CRA/RSVp-mIL-2, Surv.m-CRA/CAp-mCXCL10, and Surv.m-CRA/E2Fp-mGM-CSF (n=6) in the same manner as the method described above. After 14 days, HaK cells and HaP-T1 cells (1×10⁷ cells per group) were inoculated into the left and right dorsal regions of the hamsters, respectively. The treated animals were macroscopically observed for a further 74 days for the presence of tumor nodules after the various virus treatments. Furthermore, body weight was monitored as an indicator for evaluating side effects of these treatments.

The results are shown in Fig. 7-2 (treatment effect on the primary tumor by combination of three types of Surv.m-CRA-2), Fig. 7-3 (regression of distant-site HaK tumors by combination therapy with three types of Surv.m-CRA-2 (challenge test)), Fig. 7-4 (regression of distant-site HaP-T1 tumors by combination therapy with three types of Surv.m-CRA-2 (challenge test)), and Fig. 7-5 (changes in body weight over time), respectively.

This Example demonstrated that tumor growth can be suppressed more efficiently than with monotherapy or combination therapy of two types of cytokines by using Surv.m-CRA controlling expression of three cytokines (CXCL10, GM-CSF, and IL-2) having different mechanisms of action. In addition, this Example also revealed that the therapeutic strategy of the present invention can induce systemic antitumor immunity. Furthermore, when the therapeutic strategy of the present invention was used, poor body weight gain among treatment groups was mild, suggesting that there were no major side effects.

### [Example 8] Confirmation of expression of each gene of P2 plasmids carrying three types of therapeutic genes

Each P2 plasmid used for construction of Surv.m-CRA-2 carrying three types of therapeutic genes prepared in Example 1 (namely, (12) Surv.m-CRA/CMV-mCXCL10-mIL2-mGM (hereinafter sometimes referred to as "Surv.m-CRA/CMVp-mCIG"), (13) Surv.m-CRA/CMV-mCXCL10-mGM-mIL2 (hereinafter sometimes referred to as "Surv.m-CRA/CMVp-mCGI"), (14) Surv.m-CRA/CMV-hCXCL10-hIL2-hGM (hereinafter sometimes referred to as "Surv.m-CRA/CMVp-hCIG"), and (15) Surv.m-CRA/CMV-hCXCL10-hGM-hIL2 (hereinafter sometimes referred to as "Surv.m-CRA/CMVp-hCGI")) was transfected into HEK293 cells using the same method as in Example 2, and the expression amount of each protein was confirmed. In order to detect transgene proteins (mCXCL10, mIL-2, mGM-CSF, hCXCL10, hIL-2, hGM-CSF) *in vitro*, MILLIPLEX^{®} Mouse Cytokine/Chemokine Magnetic Bead Panel (MCYTOMAG-70K, Millipore, Burlington, MA) and MILLIPLEX^{®} Human Cytokine/Chemokine/Growth Factor Panel A (HCYTA-60K, Millipore) were performed using a Luminex-MAGPIX multiplex immunoassay system according to the manufacturer's instructions. Data were analyzed using xPONENT Software (Millipore).

As a result, expression of mouse or human CXCL10, IL-2, and GM-CSF was confirmed by transfection of P2 plasmids containing nucleic acids encoding mouse or human CXCL10, IL-2, and GM-CSF.

### [Example 9] Confirmation of expression of each gene of Surv.m-CRA-2 carrying three types of therapeutic genes

Surv.m-CRA/CMVp-mCIG, Surv.m-CRA/CMVp-mCGI, Surv.m-CRA/CMVp-hCIG, and Surv.m-CRA/CMVp-hCGI carrying three types of therapeutic genes prepared in Example 1 were used to infect HEK293 cells, and expression amounts of proteins encoded by each therapeutic gene were confirmed by ELISA.

After 48 hr from viral infection, culture supernatants were collected. Expression levels of mouse genes were examined using the MILLIPLEX^{®} Mouse Cytokine/Chemokine panel. Expression levels of human genes were measured using Human CXCL10 Quantikine ELISA Kit (R&D Systems), Human GM-CSF DuoSet ELISA (R&D Systems), and Human IL-2 Quantikine ELISA Kit (R&D Systems). As positive controls, P2 plasmids used for construction of each virus (pUni/CMVp-mCIG, pUni/CMVp-mCGI, pUni/CMVp-hCIG, pUni/CMVp-hCGI) were transfected into HEK293 cells, and supernatants collected 96 hr later were used.

As a result, in both mouse and human constructs, in CGI, the expression levels of CXCL10, GM-CSF, and IL-2 were almost equivalent (Fig. 8). On the other hand, in CIG, a higher amount of GM-CSF was detected compared with CXCL10 and IL-2 (Fig. 8, right). In any case, expression of all three factors was confirmed in all constructs.

### [Example 10] Examination of in vitro cytotoxic effect of Surv.m-CRA-2 carrying three types of therapeutic genes (WST Assay)

The cytotoxic effect on hamster-derived cancer cells (HaK and HaP-T1) and normal cells (BHK-21) infected with Surv.m-CRA/CMVp-mCGI or Surv.m-CRA/CMVp-mCIG was examined by viable cell number measurement. On the previous day, each cell type was seeded in a 96-well plate at 5×10³ cells/well. After infection with Ad.dE1.3 (Control), Surv.m-CRA (No transgene), Surv.m-CRA/CMVp-mCGI, or Surv.m-CRA/CMVp-mCIG at MOI 3 for 1 hr, culture was performed. Cytotoxicity was evaluated by WST-8 assay using Cell Count Reagent SF (Nacalai Tesque) at 3 and 5 days after infection. The experimental protocol is shown in Fig. 9-1.

As a result, in HaK and HaP-T1, Surv.m-CRA (No transgene), Surv.m-CRA/CMVp-mCGI, and Surv.m-CRA/CMVp-mCIG all showed significant cytotoxic effects compared with the control (Fig. 9-2). On the other hand, in BHK-21, little cytotoxic effect was observed with Surv.m-CRA (No transgene) and Surv.m-CRA/CMVp-mCGI, whereas a cytotoxic effect was observed with Surv.m-CRA/CMVp-mCIG (Fig. 9-2).

### [Example 11] Examination of therapeutic effect of Surv.m-CRA-2 carrying three types of therapeutic genes

In this Example, the *in vivo* treatment effect of Surv.m-CRA-2 carrying three types of therapeutic genes is confirmed. Using Surv.m-CRA-2 carrying three types of therapeutic genes, and otherwise using the same method as in Example 7 described above, examination is conducted regarding the treatment effect on the primary tumor and the induction of primary tumor-specific systemic antitumor immunity by a distant site challenge test in a Syrian hamster subcutaneous tumor model.

When three types of OVIs carrying different genes are administered, the three genes are not necessarily introduced into one cell at 100%. In contrast, when one type of OVI carrying the three genes in one virus is administered, the three types of genes are certainly introduced into one cell. Therefore, administration of one virus carrying three genes may enhance the treatment effect compared with administration of three different types of OVIs. Furthermore, administration of one type of virus carrying the three genes simultaneously is expected to improve safety because the dose can be reduced compared with administration of three types of OVIs. Since Example 9 confirmed that Surv.m-CRA-2 carrying the three genes simultaneously correctly expresses the proteins encoded by each therapeutic gene, and Example 10 confirmed the *in vitro* cytotoxic effect on cancer cells, treatment with Surv.m-CRA-2 carrying these three genes simultaneously can be expected to provide therapeutic efficacy and safety equivalent to or greater than the treatment effect shown in Example 7.

### (1) Tumor growth suppressive effect at the primary lesion

1×10⁷ HaK cells were subcutaneously transplanted into one dorsal site of 6-week-old female Syrian hamsters. At that time, HaK cells were prepared in Coring^{®} Matrigel Basement Membrane Matrix (Coring) so as to be 1×10⁷ cells/200 µL in 50% Matrigel. Approximately 14 days later, when the diameter of the transplanted tumor reached 6-10 mm, each virus of Surv.m-CRA (No transgene) or Surv.m-CRA/CMVp-mCGI (both adjusted to 1.5×10⁹ PFU/100 µL in PBS), or a virus mixture of Surv.m-CRA/CAp-mCXCL10, Surv.m-CRA/E2Fp-mGM-CSF, and Surv.m-CRA/RSVp-mIL-2 (adjusted in PBS to 5×10⁸ PFU/100 µL each, total 1.5×10⁹ PFU/100 µL), was each injected intratumorally once, and changes in tumor diameter over time were evaluated. The numbers of animals in the groups administered Surv.m-CRA (No transgene), Surv.m-CRA/CAp-mCXCL10 + Surv.m-CRA/E2Fp-mGM-CSF + Surv.m-CRA/RSVp-mIL-2, and Surv.m-CRA/CMVp-mCGI were n=8, 8, and 7, respectively. The experimental protocol is shown in Fig. 10-1.

Tumor size was measured twice a week using digital calipers. Tumor volume was calculated as long axis (mm) × short axis (mm) × short axis (mm) × 0.5 (mm³). Data are shown as mean±standard error, and statistical significance among groups was tested by Student's T-test.

As a result, against the cancer cells (nodules) at the treated primary lesion, both Surv.m-CRA/CAp-mCXCL10 + Surv.m-CRA/E2Fp-mGM-CSF + Surv.m-CRA/RSVp-mIL-2 and Surv.m-CRA/CMVp-mCGI showed significant enhancement of treatment effect (tumor suppressive effect) compared with Surv.m-CRA (No transgene), which carries no therapeutic gene (#, P<0.05 vs Surv.m-CRA (No transgene)) (Fig. 10-2).

### (2) Antitumor immune effect against distant metastasis

For each hamster in (1) above, 14 days after viral injection, 7.5×10⁶ HaK and HaP-T1 cells were each prepared so as to be 7.5×10⁶ cells/100 µL in 50% Matrigel, then subcutaneously transplanted into the dorsal region, and formation of secondary transplanted tumors was evaluated 17 days later (Fig. 10-1).

The results are shown in Table 1. The tumor formation rates by HaK retransplantation in the treatment groups of Surv.m-CRA (No transgene), Surv.m-CRA/CAp-mCXCL10 + Surv.m-CRA/E2Fp-mGM-CSF + Surv.m-CRA/RSVp-mIL-2, and Surv.m-CRA/CMVp-mCGI were 88%, 50%, and 29%, respectively, whereas in HaP-T1 transplantation, tumor formation was observed in all cases. From the above results, it became clear that, in the treatment groups of Surv.m-CRA/CAp-mCXCL10 + Surv.m-CRA/E2Fp-mGM-CSF + Surv.m-CRA/RSVp-mIL-2 and Surv.m-CRA/CMVp-mCGI, specific systemic antitumor immunity was induced and metastatic cancer cells could be effectively prevented (treated). By carrying the three therapeutic genes in one virus, a higher antitumor immune effect was obtained than in the case where the genes were carried separately in individual viruses and administered as a cocktail.

**[Table 1]**

| **#** | **Virus** | **challenge parental tumor (HaK)** | **test tumor type non-parental tumor (Hap-T1)** |
|---|---|---|---|
| **1** | **Surv.m-CRA (No transgene)** | **7/8 (88%)** | **8/8 (100%)** |
| **2** | **Surv.m-CRA/ CAp-mCXCL10 + Surv.m-CRA/ E2Fp-mGM-CSF + Surv.m-CRA/ RSVp-mIL-2** | **4/8 (50%)** | **8/8 (100%)** |
| **3** | **Surv.m-CRA/ CMVp-mCGI** | **2/7 (29%)** | **7/7 (100%)** |

Also, as shown in Fig. 10-3, no body weight loss was observed in any treatment group after virus treatment, and high safety was demonstrated. Furthermore, since appetite was also maintained in the observations of the treatment groups, it was confirmed that there was no problem with safety.

### [Example 12] Examination of treatment effect of Surv.m-CRA-2 carrying three types of therapeutic genes derived from mouse or human

### (1) Tumor growth suppressive effect at the primary lesion

1×10⁷ HaK cells were subcutaneously transplanted at one site on the dorsal side of 6-week-old female Syrian hamsters. At that time, the HaK cells were prepared with Coring^{®} Matrigel Basement Membrane Matrix (Coring) so as to be 1×10⁷ cells/200 µl in 50% Matrigel. About 10 days later, when the diameter of the transplanted tumor reached 6-10 mm, each virus of Ad.dE1.3 (Control), Surv.m-CRA (No transgene), Surv.m-CRA/CMVp-mCGI, and Surv.m-CRA/CMVp-hCGI was adjusted to 1.5×10⁹ PFU/100 µl with PBS, first injected intratumorally, and temporal changes in tumor diameter were evaluated. After 17 days from the first administration, the same dose of virus was administered. The numbers of animals in each group administered Ad.dE1.3 (Control), Surv.m-CRA (No transgene), Surv.m-CRA/CMVp-mCGI, and Surv.m-CRA/CMVp-hCGI were n=8, 8, 7, and 8, respectively. The experimental protocol is shown in Fig. 11-1.

The tumor size was measured twice per week using digital calipers. Tumor volume was calculated as major axis (mm) × minor axis (mm) × minor axis (mm) × 0.5 (mm³). Data are shown as mean±standard error, and statistical significance between groups was tested by Student's T-test.

As a result, against the cancer cells (nodules) of the treated primary lesion, Surv.m-CRA (No transgene), Surv.m-CRA/CMVp-mCGI, and Surv.m-CRA/CMVp-hCGI all showed significant treatment effects (tumor suppressive effects) compared with the control (Ad.dE1.3) (*, p<0.05 vs Ad.dE1.3 (Control)) (Fig. 11-2). The two types, Surv.m-CRA/CMVp-mCGI and Surv.m-CRA/CMVp-hCGI, showed a tendency toward further enhancement of treatment effects (tumor suppressive effects) compared with Surv.m-CRA (No transgene), which does not carry a therapeutic gene. When viewed by individual animal, 17 days after the first administration, tumor regrowth was observed in all animals in the Surv.m-CRA (No transgene)-administered group, whereas complete regression of tumors was observed in 13.5% in the Surv.m-CRA/CMVp-hCGI-administered group and in 29% in the Surv.m-CRA/CMVp-mCGI-administered group (Fig. 11-3). Furthermore, 17 days after the second administration (35 days after the first administration), the tumor regrowth rates in the Surv.m-CRA (No transgene), Surv.m-CRA/CMVp-hCGI, and Surv.m-CRA/CMVp-mCGI-administered groups were 62.5%, 37.5%, and 28.6%, respectively, and compared with Surv.m-CRA not carrying therapeutic genes, Surv.m-CRA carrying three types of therapeutic genes derived from mouse or human exhibited a remarkable enhancement of treatment effects (Fig. 11-4). Although not being bound by any particular theory, one reason why Surv.m-CRA carrying mouse genes exhibited higher treatment effects than Surv.m-CRA carrying human genes is considered to be that, particularly for GM-CSF, the homology between mouse and hamster is higher than that between human and hamster.

### (2) Antitumor immune effect against distant metastasis

For each hamster in the above (1), 14 days after the first virus administration, 7.5×10⁶ HaK and HaP-T1 cells were each prepared so as to be 7.5×10⁶ cells/100 µl in 50% Matrigel, and then subcutaneously transplanted on the dorsal side. As described above, 3 days later (17 days after the first virus administration), each virus of Ad.dE1.3 (Control), Surv.m-CRA (No transgene), Surv.m-CRA/CMVp-mCGI, and Surv.m-CRA/CMVp-hCGI was adjusted to 1.5×10⁹ PFU/100 µl with PBS and injected into the tumor of the primary lesion (second administration), and formation of the secondary transplanted tumors was evaluated 14 days thereafter (Fig. 11-1).

The results are shown in Table 2. Tumor formation due to HaK re-transplantation in the Surv.m-CRA (No transgene), Surv.m-CRA/CMVp-mCGI, and Surv.m-CRA/CMVp-hCGI treatment groups was 50%, 29%, and 25%, respectively, whereas in HaP-T1 transplantation, tumor formation was observed in all cases. From the above results, in the therapeutic gene-carrying virus administration groups, release of tumor-associated antigens and immunostimulatory factors from lysed cancer cells of the primary lesion and the adjuvant-like function of the virus were enhanced by two administrations of virus compared with a single administration, and it became clear that metastatic cancer cells could be more effectively prevented (treated) than in the virus administration group not carrying therapeutic genes.

**[Table 2]**

| **#** | **Virus** | **challenge parental tumor (HaK)** | **test tumor type non-parental tumor (Hap-T1)** |
|---|---|---|---|
| **1** | **Ad. dE1.3** | **8/ 8 (100%)** | **8/ 8 (100%)** |
| **2** | **Surv.m-CRA (No transgene)** | **4/ 8 (50%)** | **8/ 8 (100%)** |
| **3** | **Surv.m-CRA/CMVp-hCGI** | **2/ 8 (25%)** | **8/ 8 (100%)** |
| **4** | **Surv.m-CRA/CMVp-mCGI** | **2/ 7 (29%)** | **7/ 7 (100%)** |

### [Industrial Applicability]

The OVI of the present invention, in addition to having an enhanced tumor growth suppressive effect at the primary lesion compared with conventional OVI, has a remarkably enhanced ability to induce systemic primary cancer-specific antitumor immunity, and therefore can exhibit superior treatment effects even against invasive and metastatic refractory cancers for which cancer virus therapy including conventional OVI and other existing cancer treatments are ineffective. As such, the present invention is extremely useful.

This application is based on a patent application No. 2024-7682 filed in Japan (filing date: January 22, 2024), the contents of which are incorporated in full herein.

## Claims

1. An oncolytic virus comprising a nucleic acid encoding CXC motif chemokine ligand 10 (CXCL10) under the control of a promoter functional in cancer cells.

2. The oncolytic virus according to claim 1, wherein the promoter is a ubiquitous promoter, a cancer cell-specific promoter, or an organ-specific promoter of the organ from which the cancer cells are derived.

3. The oncolytic virus according to claim 1, wherein the promoter is a CA promoter, a CMV promoter, an RSV promoter, or an E2F promoter.

4. The oncolytic virus according to claim 1, wherein the promoter of a nucleic acid encoding at least one factor essential for viral replication or assembly is substituted with a cancer cell-specific promoter or an organ-specific promoter of the organ from which the cancer cells are derived.

5. The oncolytic virus according to claim 4, wherein the cancer cell-specific promoter is a survivin promoter.

6. The oncolytic virus according to claim 4, wherein the virus is an adenovirus.

7. The oncolytic virus according to claim 6, wherein the factor essential for viral replication or assembly is E1A or E1AΔ24, and/or E1B or E1BΔ55K.

8. The oncolytic virus according to claim 7, wherein the promoter of the nucleic acid encoding E1A is substituted with a survivin promoter, and the promoter of the nucleic acid encoding E1BΔ55K is further substituted with an exogenous promoter selected from a ubiquitous promoter, a cancer cell-specific promoter, and an organ-specific promoter of the organ from which the cancer cells are derived.

9. The oncolytic virus according to claim 8, wherein the promoter of the nucleic acid encoding E1BΔ55K is substituted with a CMV promoter.

10. The oncolytic virus according to any one of claims 1 to 9, further comprising a nucleic acid encoding interleukin-2 (IL-2) under the control of a promoter functional in cancer cells, and/or a nucleic acid encoding granulocyte-macrophage-colony-stimulating factor (GM-CSF) under the control of a promoter functional in cancer cells.

11. The oncolytic virus according to claim 1, further comprising a nucleic acid encoding interleukin-2 (IL-2) under the control of a promoter functional in cancer cells, and a nucleic acid encoding granulocyte-macrophage colony-stimulating factor (GM-CSF) under the control of a promoter functional in cancer cells.

12. The oncolytic virus according to claim 10, wherein the promoter controlling expression of CXCL10 and the promoter controlling expression of IL-2 and/or the promoter controlling expression of GM-CSF are the same single promoter.

13. The oncolytic virus according to claim 12, wherein the nucleic acid encoding CXCL10 and the nucleic acid encoding IL-2 and/or the nucleic acid encoding GM-CSF are arranged in the following order from the 5' end:
(i) CXCL10-IL-2;
(ii) CXCL10-GM-CSF;
(iii) IL-2-CXCL10;
(iv) GM-CSF-CXCL10;
(v) CXCL10-IL-2-GM-CSF;
(vi) CXCL10-GM-CSF-IL-2;
(vii) IL-2-CXCL10-GM-CSF;
(viii) IL-2-GM-CSF-CXCL10;
(ix) GM-CSF-CXCL10-IL-2; or
(x) GM-CSF-IL-2-CXCL10.

14. The oncolytic virus according to claim 13, wherein each of the nucleic acids is linked via a 2A sequence or an IRES sequence.

15. The oncolytic virus according to claim 14, wherein, from the 5' end, the first nucleic acid and the second nucleic acid are linked via a P2A sequence, and the second nucleic acid and the third nucleic acid are linked via a T2A sequence.

16. A combination of the oncolytic virus according to any one of claims 1 to 9 and the oncolytic viruses of (a) and/or (b), or the oncolytic virus of (c) below:
(a) an oncolytic virus comprising a nucleic acid encoding IL-2 under the control of a promoter functional in cancer cells
(b) an oncolytic virus comprising a nucleic acid encoding GM-CSF under the control of a promoter functional in cancer cells
(c) an oncolytic virus comprising a nucleic acid encoding IL-2 under the control of a promoter functional in cancer cells, and a nucleic acid encoding GM-CSF under the control of a promoter functional in cancer cells.

17. The combination according to claim 16, wherein the promoters functional in cancer cells are the same or different, and each is a ubiquitous promoter, a cancer cell-specific promoter, or an organ-specific promoter of the organ from which the cancer cells are derived.

18. The combination according to claim 16, wherein the promoters functional in cancer cells are identical or different, and each is a CA promoter, a CMV promoter, an RSV promoter, or an E2F promoter.

19. The combination according to claim 16, wherein the oncolytic virus is a conditionally replicating adenovirus, and the structure of the replication control region of each oncolytic virus is identical.

20. A cancer therapeutic agent comprising the oncolytic virus according to any one of claims 1 to 9 as an active ingredient.

21. The agent according to claim 20, for local administration to a primary cancer lesion.

22. The agent according to claim 20, for the treatment of invasive or metastatic cancer.

23. The agent according to claim 20, for multiple administration.

24. A cancer therapeutic agent comprising the combination according to claim 16 as an active ingredient.

25. The agent according to claim 24, for local administration to a primary cancer lesion.

26. The agent according to claim 24, for the treatment of invasive or metastatic cancer.

27. The agent according to claim 24, for multiple administration.
